(19) Europäisches Patentamt / European Patent Office / Office européen des brevets

(11) **EP 2 784 493 B1**

(12) **EUROPEAN PATENT SPECIFICATION**

(45) Date of publication and mention of the grant of the patent:
**04.01.2017 Bulletin 2017/01**

(21) Application number: **12851197.9**

(22) Date of filing: **20.11.2012**

(51) Int Cl.:
***G01N 27/04*** *(2006.01)* ***G01N 27/12*** *(2006.01)*
***G01N 27/327*** *(2006.01)* ***G01N 33/483*** *(2006.01)*
***G01N 33/543*** *(2006.01)*

(86) International application number:
**PCT/JP2012/007425**

(87) International publication number:
**WO 2013/076959 (30.05.2013 Gazette 2013/22)**

(54) **MEASURING METHOD FOR BIOLOGICAL SUBSTANCE AND MEASURING DEVICE THEREFOR**

MESSVERFAHREN FÜR BIOLOGISCHE SUBSTANZEN UND MESSVORRICHTUNG DAFÜR

PROCÉDÉ DE MESURE POUR SUBSTANCE BIOLOGIQUE ET DISPOSITIF DE MESURE POUR CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2011 JP 2011257037**

(43) Date of publication of application:
**01.10.2014 Bulletin 2014/40**

(73) Proprietor: **Panasonic Intellectual Property Management Co., Ltd.**
**Osaka 540-6207 (JP)**

(72) Inventors:
- **NOMURA, Takaiki**
  **2-1-61 Shiromi, Chuo-ku, Osaka 540-6207 (JP)**
- **NUKINA, Yasuyuki**
  **2-1-61 Shiromi, Chuo-ku, Osaka 540-6207 (JP)**
- **HATO, Kazuhito**
  **2-1-61 Shiromi, Chuo-ku, Osaka 540-6207 (JP)**
- **TOKUMITSU, Shuzo**
  **2-1-61 Shiromi, Chuo-ku, Osaka 540-6207 (JP)**

(74) Representative: **Müller-Boré & Partner Patentanwälte PartG mbB**
**Friedenheimer Brücke 21**
**80639 München (DE)**

(56) References cited:
**JP-A- 2002 174 614** **JP-A- 2002 333 419**
**JP-A- 2003 254 978** **JP-A- 2003 262 601**
**JP-A- 2004 108 813** **JP-A- 2004 108 814**

- **S. KOBAYASHI ET AL: "Control of carrier density by self-assembled monolayers in organic field-effect transistors", NATURE MATERIALS, vol. 3, no. 5, 4 April 2004 (2004-04-04), pages 317-322, XP055006632, ISSN: 1476-1122, DOI: 10.1038/nmat1105**
- **PERNSTICH K ET AL: "Threshold voltage shift in organic field effect transistors by dipole monolayers on the gate insulator", JOURNAL OF APPLIED PHYSICS, AMERICAN INSTITUTE OF PHYSICS, US, vol. 96, no. 11, 1 January 2004 (2004-01-01), pages 6431-6438, XP012068321, ISSN: 0021-8979, DOI: 10.1063/1.1810205**
- **PING TONG ET AL: "Label-free immunosensing of microcystin-LR using a gold electrode modified with gold nanoparticles", MICROCHIMICA ACTA ; AN INTERNATIONAL JOURNAL ON MICRO AND TRACEANALYSIS, SPRINGER-VERLAG, VI, vol. 173, no. 3 - 4, 19 February 2011 (2011-02-19), pages 299-305, XP019901129, ISSN: 1436-5073, DOI: 10.1007/S00604-011-0557-8**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 784 493 B1

## Description

TECHNICAL FIELD

The present invention relates to a method, a device and a system for analyzing or measuring a biological substance (for example, an antigen or antibody) electrochemically.

BACKGROUND ART

For a method for specifically detecting a biological substance, an antigen-antibody reaction or an immunological reaction has been used. For example, an ABO blood determination using no marker and many detecting means using a radioisotope and a fluorescent material as markers and a redox current based on an enzyme have been developed.
However, these methods are methods of detecting, for one molecule of a selected biological substance, one atom of an isotope, or several electrons or similar-level electrons that contribute to redox. Thus, the detection is very difficult.
On the other hand, the recognition of a neurotransmitter, acetylcholine, and a response thereto through an acetylcholine receptor in a neural synapse are known as a typical example for detecting a substance in vivo. The acetylcholine receptor penetrates through a lipid bilayer to be present in the state of directing its acetylcholine recognizing site outward. The recognition of acetylcholine for the acetylcholine receptor and the binding of acetylcholine to the acetylcholine receptor are specific to be high in precision and sensitivity. The, acetylcholine receptor which recognizes acetylcholine and binds to acetylcholine changes the conformation to penetrate through a lipid bilayer so that an ion channel is formed. Usually, the inside and the outside of any lipid bilayer are in an electrically polarized state based on an uneven distribution of ions. However, through the ion channel, the ions are transferred and mixed with each other so that depolarization is induced. This depolarization state is transferred as a pulse in nerve cells so that information is transmitted to the site apart from the original site in a living body (neural transmission). As shown by this example, the detection of a substance in vivo is high in precision and sensitivity, and further, recognized information is converted into electric signals through a simple and rapid one-step process, and subsequent information processing is attained. In this point, the detection is a very reasonable detection method.
An immunological electrode method which has both of the versatility of an immunological reaction and advantages seen in the sensing of a biological receptor, such as sensitivity, precision, simplicity and rapidness is reported where an immunological reaction is measured as an electric response (Patent Documents 1 and 2).

PRIOR ART DOCUMENTS

PATENT DOCUMENTS

Patent Document 1: Japanese Patent Laid-open Publication No. 2002-174612
Patent Document 2: Japanese Patent Laid-open Publication No. 2002-333419

SUMMARY OF THE INVENTION

PROBLEMS TO BE SOLVED BY THE INVENTION

When a lipid membrane as observed in a biological membrane is formed on an electrode, an ion channel may not reach onto the electrode because the membrane is too thick or the molecules are stacked onto each other at random. Moreover, the lipid membrane undergoes hydrolysis etc., so that its sensing moiety may be deteriorated with time.
Thus, the inventors have made further developments to employ, instead of a lipid membrane, a thin film containing a hydrocarbon group, preferably a monomolecular film containing the same to achieve an invention regarding an immunological electrode method having good electric responsibility and reliability.
The present specification provides an immunological electrode method having good electric responsibility and reliability, a device using this method, and others.

SOLUTIONS TO THE PROBLEMS

A first aspect of the present invention provides a device or chip for measuring the amount of a biological substance in a liquid to be measured, comprising:

an immobilized antibody; a substance labeled with an ion conductive compound wherein the substance binds to the immobilized antibody; and

metal electrodes comprising a working electrode and a counter electrode, wherein the working electrode has, on its surface, a thin film comprising a linear fluoroalkyl group and the number of carbon atoms of the linear fluoroalkyl group is from 3 to 12.

These aspects of the present invention may each be realized by a system, a method and any combination thereof.

EFFECTS OF THE INVENTION

Use of a hydrocarbon group for a thin film improves endurance against deterioration based on use, and thereby, makes it possible to provide a measuring method for a biological substance, a measuring chip therefor and a measuring device therefor that are improved in electric responsibility and reliability.

BRIEF DESCRIPTION OF THE DRAWINGS

Fig. 1 illustrates a schematic view of a mechanism for detecting a biological substance.
Fig. 2 illustrates a schematic view of a mechanism for detecting an antigen.
Fig. 3 illustrates a synthesis scheme of a labeled antigen.
Fig. 4 illustrates a synthesis scheme of an immobilized antibody.
Fig. 5 illustrates a synthesis scheme of a monoclonal antibody.
Fig. 6 illustrates a schematic view of a mechanism for detecting an antibody.
Fig. 7 illustrates a synthesis scheme of an immobilized antigen.
Fig. 8 illustrates a synthesis scheme of a labeled antibody.
Fig. 9 illustrates a measuring chip and device for an antigen.
Fig. 10 illustrates a measuring chip and device for an antibody.
Fig. 11 shows an electric conductivity response of an estradiol antigen versus the concentration thereof.
Fig. 12 shows an electric conductivity response (obtained by axial conversion) of the estradiol antigen versus the concentration thereof.
Fig. 13 shows an electric conductivity response of an estradiol antigen versus temperature.
Fig. 14 shows an electric conductivity response (obtained by axial conversion) of the estradiol antigen versus temperature.
Fig. 15 shows an electric conductivity response of an estradiol antigen to a lipid bilayer membrane.
Fig. 16 shows an electric conductivity response (obtained by axial conversion) of the estradiol antigen to the lipid bilayer membrane.
Fig. 17 shows an electric conductivity response of an estradiol antigen to a fluoroalkyl film.
Fig. 18 shows an electric conductivity response (obtained by axial conversion) of the estradiol antigen to the fluoroalkyl film.
Fig. 19 shows an electric conductivity response of an estradiol antigen versus the length of alkyl groups.
Fig. 20 shows an electric conductivity response (obtained by axial conversion) of the estradiol antigen versus the length of the alkyl groups.
Fig. 21 shows an electric conductivity response of an estradiol antigen to an alkylsiloxane film.
Fig. 22 shows an electric conductivity response (obtained by axial conversion) of the estradiol antigen to the alkylsiloxane film.

EMBODIMENTS OF THE INVENTION

A. Chip and Device for Measuring Amount of Biological substance in Liquid. Using Immobilized antibody and Substance Labeled with Ion Conductive Compound

The first aspect of the present invention provides a device or chip for measuring the amount of a biological substance in a liquid to be measured, comprising:

an immobilized antibody; a substance labeled with an ion conductive compound wherein the substance binds to the immobilized antibody; and

metal electrodes comprising a working electrode and a counter electrode, wherein the working electrode has, on its surface, a thin film comprising a linear fluoroalkyl group and the number of carbon atoms of the linear fluoroalkyl group is from 3 to 12.

The device or chip provided by the first aspect of the invention may further comprise a section for measuring the temperature of the liquid to be measured. The chip provided by the first aspect of the invention may be integrated into a device or system for measuring the amount of the biological substance in the liquid to be measured.

In one embodiment, the biological substance in the liquid to be measured is allowed to contact with the immobilized antibody to release, from the immobilized antibody, the substance which is labeled with the ion transmissible compound and binds to the immobilized antibody; an ion transmissible compound moiety of the released substance labeled with the ion transmissible compound is adsorbed onto the thin film formed on the surface of the working electrode; the amount of a change in the electric conductivity of the thin film that is induced by the adsorption of the ion conductive compound moiety is measured; and further the temperature of the liquid to be measured is optionally measured to determine the amount of the biological substance in the liquid to be measured.

In this embodiment, the biological substance and the substance labeled with the ion conductive compound each contain the site which the immobilized antibody recognizes and then bind to. In this case, the biological substance bind to the immobilized antibody while competing with the substance labeled with the ion conductive compound.

In this embodiment, in each of the biological substance and the substance labeled with the ion conductive compound, its site which the immobilized antibody recognizes may be entirely identical with an epitope of an antigen which can be used for preparing the immobilized antibody, or may be different therefrom as far as the immobilized antibody can bind to the site. For example, in the biological substance and the substance labeled with the ion conductive compound, respective amino acid sequences of their sites which the immobilized antibody recognizes may be identical with or different from each other as far as the immobilized antibody shows cross-reactivity (the difference is, for example, one residue, two residues, three residues, four residues or five residues).

In this embodiment, at least one of the biological substance and the substance labeled with the ion conductive compound may be an antibody corresponding to the immobilized antibody. Moreover, the substance labeled with the ion conductive compound may be identical with the biological substance as a substance if the substance is not labeled with the ion conductive compound, or different from the biological substance.

In one embodiment, the biological substance in the liquid to be measured is allowed to contact with the substance which is labeled with the ion conductive compound and binds to the immobilized antibody, so as to release, from the immobilized antibody, the substance labeled with the ion transmissible compound; an ion transmissible compound moiety of the released substance labeled with the ion transmissible compound is adsorbed onto the thin film formed on the surface of the working electrode; the amount of a change in the electric conductivity of the thin film that is induced by the adsorption of the ion conductive compound moiety is measured; and further the temperature of the liquid to be measured is optionally measured to determine the amount of the biological substance in the liquid to be measured.

In this embodiment, the biological substance may be an antibody which recognizes the substance labeled with the ion conductive compound. In this way, the biological substance binds to the substance labeled with the ion conductive compound while competing with the immobilized antibody.

In this embodiment, the site of the substance labeled with the ion transmissible compound which the biological substance recognizes may be entirely identical with o the recognition site of the substance labeled with the ion transmissible compound which the immobilized antibody recognizes or different from the recognition site as far as the substance labeled with the ion transmissible compound can bind to the sites. For example, in the site of the substance labeled with the ion transmissible compound which the biological substance recognizes, and the recognition site labeled with the ion transmissible compound which the immobilized antibody recognizes, respective amino acid sequences of these sites may be identical with or different from each other as far as the both antibodies bind thereto (the difference is, for example, one residue, two residues, three residues, four residues or five residues).

In this embodiment, the substance labeled with the ion conductive compound may be an antigen corresponding to at least one of the immobilized antibody and the biological substance. The immobilized antibody may be identical with or different from the biological substance as a substance if the immobilized antibody is not immobilized.

In the present specification, the biological substance is a substance derived from any living body, and may be a physiologically active substance. Examples thereof include low molecular substances derived from an animal (for example, the human) (such as estradiol), and proteins (such as hemoglobin, and hCG (human chorionic gonadotropin), and antigens). Other examples of the biological substance include cancer antigens (such as AFP (alpha-fetoproten), BCA 225, BFP (basic fetoprotein), CEA (carcinoembryonic antigen), CA 15-3 (carbohydrate antigen 15-3), CA 125 (carbohydrate antigen 125), CA 54/61 (CA 546, carbohydrate antigen 54/61), CA 19-9 (carbohydrate antigen 19-9), CA 72-4 (carbohydrate antigen 72-4), DUPAN-2 (pancreatic cancer associated antigen), elastase 1, ferritin, IAP (immunosuppressive acidic protein), KM 01, NSE (neutron-specific enolase), NCC-ST-439, urinary polyamine, PIVKA-II (abnormal prothrombin, protein induced by vitamin K absence-2), PA (prostate specific antigen), PAP (prostatic acid phosphatase),

SCC (squamous cell carucinoma-related antigen), sialyl SSEA-1 antigen, TPA (tissue polypeptide antigen), and γ-Sm (gamma-seminoprotein). A substance known already as a substance detectable by the use of an antigen-antibody reaction can be a biological substance measurable by the present invention.

In the specification, the biological substance may be contained in a liquid to be measured. In the specification, the liquid to be measured is a liquid in which a substance to be measured is contained. Examples thereof include a buffer solution, saline, body fluids (such as blood and urine), and liquids prepared from a living body (such as serum and plasma).

In the specification, the chip is not particularly limited, and may be a small packaged constituent unit, function unit, or device. The chip may be, for example, an electrochemical sensor or electrode sensor for detecting an electric change based on a chemical substance such as ions (such as sodium ions).

In the specification, the immobilized antibody may be an antibody immobilized onto a substrate. In the specification, the substrate may be a support for a solid-state material, and may be made of a polymer such as polypropylene, polyethylene, polystyrene, polydimethylsiloxane, or polystyrene. The shape thereof is not particularly limited, and may be the shape of beads or a plate. For immobilizing an antibody onto the substrate, a method which can be ordinarily used by those skilled in the art may be used. For example, an antibody may be allowed to be directly adsorbed onto a substrate of polystyrene, or may be immobilized indirectly through a linker onto a substrate (see Japanese Patent Laid-open Publication No. 11-322799, and others). Such a linker is commercially available from Pias Corporation, and, for example, sulfo-SMCC may be used.

In the specification, the antibody is not particularly limited, and may be, for example, IgG, IgM, IgA, IgE, or IgD. The antibody is preferably a monoclonal antibody. The antibody may be a monovalent antibody. Examples of the antibody which can be used for the present invention include a monoclonal antibody binding specifically to estradiol, a monoclonal antibody binding specifically to hemoglobin, a monoclonal antibody binding specifically to hCG (human chorionic gonadotropin), and respective monoclonal antibodies binding specifically to the above-mentioned various cancer antigens.

In one embodiment, the antibody used to prepare the immobilized antibody recognizes a biological substance as an antigen. In other words, in the embodiment, a biological substance is an antigen corresponding to the antibody used to prepare the immobilized antibody.

In one embodiment, the antibody used to prepare the immobilized antibody is a monovalent antibody.

In this embodiment, a reaction between the biological substance or the substance labeled with the ion conductive compound, and the immobilized antibody is limited only to a one-to-one reaction. Thus, the embodiment is excellent in quantitativity.

In one embodiment, the antibody used to prepare the immobilized antibody is a monoclonal antibody.

In this embodiment, the affinity between the biological substance or the substance labeled with the ion conductive compound, and the immobilized antibody is uniform. Thus, the embodiment is excellent in quantitativity.

In the specification, the substance labeled with the ion conductive compound is not particularly limited, and may be a substance labeled using a compound having an ion transmissible group. In the specification, the ion transmissible group is not particularly limited, and may be a functional group for forming a channel for ions (such as sodium ions or potassium ions).

In one embodiment, the ion conductive group may be a group having a cyclic structure having an orientation structure having large inward polarity and small outward polarity. Examples of the ion conductive group include a crown ether group, a group having porphyrin, and a group having phthalocyanine. Examples of the crown ether group used in the present invention include groups each having a crown ether having a structure represented by $(-CH_2-CH_2-O-)_n$ wherein n is 5 or more (for example, 15-crown-5, 18-crown-6, dibenzo-18-crown-6, and diaza-18-crown-6).

In this embodiment, the biological substance labeled with the ion conductive group, which ion conductive group has a cyclic structure having an orientation structure having large inward polarity and small outward polarity, is adsorbed onto the thin film formed on one (the working electrode) of the electrodes and containing the hydrocarbon group. The outward moiety of the hydrophobic cyclic structure attracts the hydrophobic hydrocarbon group through hydrophobic bonding, whereby the ion conductive group penetrates through the hydrocarbon group-containing thin film so that a channel is formed up to the upper of the electrode, and further ions are easily introduced into the region of the channel by the inner moiety of the cyclic structure. As a result, based on a change in the electric conductivity of the thin film, the biological substance labeled with the ion conductive group can be detected.

In one embodiment, the cyclic structure of the ion conductive group has a size equal to or more than such a size that a sodium ion can undergo clathration.

In this embodiment, sodium ions contained in the liquid to be measured (for example, a liquid originating from a living body, a buffer solution or a physiological salt solution) can easily move inside the ion channel so that the electric responsibility is improved.

Examples of the compound having an ion transmissible group (i.e., the ion conductive compound) include, but are not particularly limited to, a compound having the following formula:

[Chem. 1]

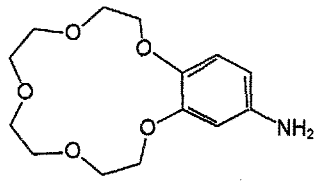

Other examples of the ion conductive compound include valinomycin, L-alanine and others. The substance labeled with the ion conductive compound is not particularly limited, and examples thereof include low molecular substances derived from an animal (for example, the human) (such as estradiol), and proteins (such as hemoglobin, and hCG (human chorionic gonadotropin), and antigens). Other examples of the substance labeled with the ion conductive compound include cancer antigens, examples of which include the same examples as described regarding the biological substance.

The labeling of the substance with the ion transmissible compound can be appropriately performed by those skilled in the art. For example, the ion transmissible compound, or its ion transmissible group may be bound directly or indirectly through, for example, a linker (through an amide bond, ester bond or ether bond) to the substance. For example, the substance labeled with the ion transmissible compound may be produced by causing an amino group of the ion transmissible compound or its ion conductive group to react with a carboxyl group of the substance to form an amide.

In the specification, the electrodes may be composed of a pair of two electrodes, and may be composed of, for example, a working electrode, which is an electrode on which an electrochemical reaction occurs, and a counter electrode, which is a reference electrode when a voltage is applied to between the electrodes. The electrodes may be made of a metal such as gold, platinum or silver, or a metal oxide such as ITO (indium tin oxide), Snow, ZnO, or TiOz. The surface of the metal or metal oxide of the working electrode may be coated with a thin film containing a hydrocarbon group.

In the specification, the thin film containing a hydrocarbon group may be a thin film constituted by a chemical substance having a hydrocarbon group.

In one embodiment, the thin film containing a hydrocarbon group is produced, for example, by reacting a metal with a hydrocarbon group having, at a terminal thereof, a group that can be bound to the metal, such as sulfide, diselenide, selenide, thiol, nitrile, isonitrile, nitro, selenol, a trivalent phosphorous compound, isothiocyanate, xanthate, thiocarbate, phosphine, thioacid, or dithioacid, to bind the group to the metal.

The hydrocarbon group is, a linear fluoroalkyl group, or a linear fluoroalkyl group containing a siloxane in each of which the alkyl group is a saturated or unsaturated alkyl group. The number of carbon atoms in each of these groups may be 1, 2, 3, 4, 5, 6, 7, 8, 9, 10, 11 or 12, and is, for example, 2, 3 or 4. Examples of these groups include $CF_3(CF_2)_{n-2}(CH_2)_2$- wherein n is 2, 3, 4, 5, 6, 7, 8, 9, 10 or 11, or 11, and $CF_3(CF_2)_{n-2}(CH_2)_2$-Si- wherein n is 2, 3, 4, 5, 6, 7, 8, 9, 10 or 11.

In this embodiment, the substance labeled with the ion conductive compound is adsorbed onto the thin film formed on one (the working electrode) of the electrodes and containing the linear alkyl group, whereby an ion channel is formed in a substantially linear form from the ion conductive group up to the upper of the electrode while penetrating through the thin film containing the linear alkyl group. Moreover, the deterioration of the thin film containing the linear alkyl group with time is restrained. It is therefore possible to provide a measuring method, a measuring chip and a measuring device for a substance that are each remarkably improved in electric responsibility and reliability. These advantageous effects can be made more remarkable by converting the group to a fluoroalkyl group since rigidity and stability of the molecular chain is increased.

In one embodiment, the thin film may be a monomolecular film.

In this embodiment, the substance labeled with the ion conductive compound is adsorbed onto the monomolecular film formed on one (the working electrode) of the electrodes and containing the hydrocarbon group so that no molecules are laminated at random. Thus, an ion channel is formed in a substantially linear form from the ion conductive group up to the upper of the electrode while penetrating through the monomolecular film. It is therefore possible to provide a measuring method, a measuring chip and a measuring device for a substance that are each further remarkably improved in electric responsibility and reliability.

The section for measuring the temperature of the liquid to be measured is not particularly limited as far as the section has a function of measuring the temperature of the liquid to be measured. The section for measuring the temperature of the liquid to be measured may be, for example, a temperature sensor.

The section for measuring the temperature of the liquid to be measured may be set at any position of the device or chip as far as the section makes it possible to measure the temperature of the liquid to be measured. In one embodiment, the section for measuring the temperature of the liquid to be measured may be set to measure the temperature of the liquid on the front surface of the thin film containing the hydrocarbon group.

In one embodiment, the device or chip provided by the present invention may further have an electric conductivity detecting section and a substance concentration calculating section. It is sufficient that the electric conductivity detecting section can measure the amount of a change in the electric conductivity of the thin film that is induced by the adsorption of the ion conductive compound moiety. This section may be an electric conductivity meter. Since the change in the electric conductivity is correlative with the amount of the adsorbed ion conductive compound moiety, the amount of the biological substance in the liquid to be measured is measured in the substance concentration calculating section.

In one embodiment, in the device or chip provided by the present invention, a single channel constitutes a structure from a liquid inputting opening through which the liquid to be measured is supplied to a discharging opening through which the liquid subjected to the measurement is discharged. This structure has, in turn from the inputting opening of the liquid to be measured and on a surface of the channel, (1) the immobilized antibody to which the substance labeled with the ion conductive compound binds, and (2) the working electrode having, on the surface thereof, the thin film containing the hydrocarbon group.

In one embodiment, the section for measuring the temperature of the liquid to be measured may be set in the channel between (1) the immobilized antibody to which the substance labeled with the ion conductive compound binds, and (2) the working electrode having, on the surface thereof, the thin film containing the hydrocarbon group. In one embodiment, a stirrer for stirring for making the solution temperature even may be set inside the channel. In one embodiment, the liquid to be measured is an even solution flow at the pipe cavity from the inputting opening to the discharging opening.

In one embodiment, the device or chip provided by the present invention may be designed in such a manner that the substrate on which the immobilized antibody is immobilized, the electrode, and the other portion can be separated from each other, and any one thereof can be replaced. For example, the substrate portion on which the immobilized antibody of the device or chip provided by the invention is immobilized may be designed to be replaced with a substrate on which a different immobilized antibody is immobilized.

In one embodiment, before the liquid to be measured contacts the substance labeled with the ion transmissible compound, the substance is binding to the immobilized antibody. In another embodiment, the substance labeled with the ion conductive compound is not binding to the immobilized antibody before the liquid to be measured contacts the substance. In this embodiment, the substance labeled with the ion transmissible compound is brought into contact with the immobilized antibody, simultaneously with the liquid to be measured or in the condition where the substance is beforehand mixed with the liquid to be measured.

<u>B. Chip and Device for Measuring Amount of Biological substance in Liquid. Using Immobilized Substance and Antibody Labeled with Ion Conductive Substance</u>

Is is disclosed a device or chip for measuring the amount of a biological substance in a liquid to be measured, comprising:

> an immobilized substance; an antibody labeled with an ion conductive compound, wherein the antibody binds to the immobilized substance; and electrodes comprising a working electrode and a counter electrode, wherein the working electrode has, on its surface, a thin film comprising a hydrocarbon group.

The device or chip provided may further comprise a section for measuring the temperature of the liquid to be measured. The chip may be integrated into a device or system for measuring the amount of the biological substance in the liquid to be measured.

In one embodiment, the biological substance in the liquid to be measured is allowed to contact the immobilized substance to release, from the immobilized substance; the antibody which is labeled with the ion transmissible compound and binds to the immobilized substance; an ion transmissible compound moiety of the released antibody labeled with the ion transmissible compound is adsorbed onto the thin film formed on the surface of the working electrode; the amount of a change in the electric conductivity of the thin film that is induced by the adsorption of the ion conductive compound moiety is measured; and further the temperature of the liquid to be measured is optionally measured to determine the amount of the biological substance in the liquid to be measured.

In this embodiment, the biological substance may be an antibody, and the antibody, which is the biological substance, and the antibody labeled with the ion conductive compound each recognize the immobilized substance to bind to this substance. In this case, the antibody, which is the biological substance, binds to the immobilized substance while competing with the antibody labeled with the ion conductive compound.

In this embodiment, sites where the antibody, which is the biological substance, and the antibody labeled with the ion conductive compound each recognize the immobilized substance may be entirely identical with or different from each other as far as the sites can bind to the immobilized substance. For example, in the antibody, which is the biological substance, and the antibody labeled with the ion conductive compound, respective amino acid sequences of their

immobilized substance-recognizing sites may be identical with, or may be different from each other as far as the sites can bind to the immobilized substance (the difference is, for example, one residue, two residues, three residues, four residues or five residues).

In this embodiment, the immobilized substance may be an antigen corresponding to at least one of the biological substance and the antibody labeled with the ion conductive compound. The antibody labeled with the ion conductive compound may be identical with the biological substance as a substance if the antibody is not being labeled with the ion conductive compound or different from the biological substance as a substance.

In one embodiment, the biological substance in the liquid to be measured is allowed to contact the antibody which is labeled with the ion conductive compound and binds to the immobilized substance, so as to release, from the immobilized substance, the antibody labeled with the ion transmissible compound; an ion transmissible compound moiety of the released substance labeled with the ion transmissible compound is adsorbed onto the thin film formed on the surface of the working electrode; the amount of a change in the electric conductivity of the thin film that is induced by the adsorption of the ion conductive compound moiety is measured; and further the temperature of the liquid to be measured is optionally measured to determine the amount of the biological substance in the liquid to be measured.

In this embodiment, the biological substance may be a substance which binds to the antibody labeled with the ion conductive compound, and is, for example, a substance that may become an antigen corresponding to the antibody labeled with the ion conductive compound. In this way, the biological substance binds to the antibody labeled with the ion conductive compound while competing with the immobilized substance.

In this embodiment, the site of the biological substance which the antibody labeled with the ion transmissible compound recognizes may be entirely identical with the recognition site of the immobilized substance which the antibody labeled with the ion transmissible compound recognizes or different from the recognition site as far as the antibody labeled with the ion transmissible compound can bind to the sites. For example, in the site of the biological substance which the antibody labeled with the ion transmissible compound recognizes, and the recognition site of the immobilized substance which the antibody labeled with the ion transmissible compound recognizes, respective amino acid sequences of these sites may be identical with or different from each other as far as the both antibodies bind thereto (the difference is, for example, one residue, two residues, three residues, four residues or five residues).

In this embodiment, at least one of the immobilized substance and the biological substance may be an antigen corresponding to the antibody labeled with the ion transmissible compound. The immobilized substance may be identical with or different from the biological substance as a substance if the immobilized substance is not being immobilized.

In the present specification, the immobilized substance may be a substance immobilized on a substrate. The substance is not particularly limited, and examples thereof include low molecular substances originating from an animal (for example, the human) (such as estradiol), and proteins (such as hemoglobin, and hCG (human chorionic gonadotropin), and antigens). Other examples of the substance that can be immobilized on the substrate include cancer antigens, examples of which include the same examples as described regarding the biological substance in the above-mentioned item A. A substance known already as a substance detectable by the use of an antigen-antibody reaction may be an immobilized substance.

In the specification, the substrate may be a support for a solid-state material, and may be made of a polymer such as polypropylene, polyethylene, polystyrene, polydimethylsiloxane, or polystyrene. The shape thereof is not particularly limited, and may be the shape of beads or a plate. For immobilizing a substance onto the substrate, a method which can be ordinarily used by those skilled in the art may be used. For example, an antibody may be allowed to be directly adsorbed onto a substrate of polystyrene, or may be immobilized indirectly through a linker onto a substrate (see Japanese Patent Laid-open Publication No. 11-322799, and others). Such a linker is commercially available from Pias Corporation, and is, for example, sulfo-SMCC. In one embodiment, the immobilized substance may be estradiol binding to polystyrene in which disuccinyldisulfide is used as a linker, as represented by the following formula:

[Chem. 2]

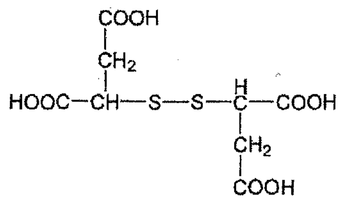

The ion transmissible compound and the antibody used in the antibody labeled with the ion conductive compound are as described in the item A. The method for labeling the antibody with the ion transmissible compound can also be performed by those skilled in the art with reference to the description in the item A.

Other terms, for example, the following terms are as described in the item A: electrodes including a working electrode and a counter electrode, section for measuring the temperature of the liquid to be measured, electric conductivity detecting section, substance concentration calculating section, hydrocarbon group, thin film, chip and others. Any constituent that can be used in the device or chip provided by the first aspect of the present invention can be used in the device or chip provided by the second aspect of the invention.

In one embodiment, in the device or chip provided by the present invention, a single channel constitutes a structure from a liquid to be measured-inputting opening through which the liquid to be measured is supplied to a discharging opening through which the liquid subjected to the measurement is discharged. This structure has, in turn from the inputting opening of the liquid to be measured and on a surface of the channel, (1) the immobilized substance to which the antibody labeled with the ion conductive compound binds, and (2) the working electrode having, on the surface thereof, the thin film containing the hydrocarbon group.

In one embodiment, the section for measuring the temperature of the liquid to be measured may be set in the channel between (1) the immobilized antibody to which the substance labeled with the ion conductive compound binds, and (2) the working electrode having, on the surface thereof, the thin film containing the hydrocarbon group. In one embodiment, a stirrer for stirring for making the solution temperature even may be set inside the channel. In one embodiment, the liquid to be measured is an even solution flow at the pipe cavity from the inputting opening to the discharging opening.

In one embodiment, the device or chip provided by the present invention may be designed in such a manner that the substrate on which the immobilized substance is immobilized, the electrode, and the other portion can be separated from each other, and any one thereof can be replaced. For example, the substrate portion on which the immobilized substance of the device or chip provided by the invention is immobilized may be designed to be replaced with a substrate on which a different immobilized substance is immobilized.

In one embodiment, before the liquid to be measured contacts the antibody labeled with the ion transmissible compound, this antibody is binding to the immobilized substance. In another embodiment, the antibody labeled with the ion conductive compound is not binding to the immobilized substance before the liquid to be measured contacts the antibody. In this embodiment, the antibody labeled with the ion transmissible compound is brought into contact with the immobilized substance, simultaneously with the liquid to be measured or in the condition where the antibody is beforehand mixed with the liquid to be measured.

<u>C. Method for Measuring Amount of Biological substance, Using Immobilized Antibody and Substance Labeled with Ion Conductive Substance</u>

A third aspect of the present invention provides a method for measuring the amount of a biological substance in a liquid to be measured, comprising: allowing an immobilized antibody which binds to a substance labeled with an ion conductive compound to contact with the biological substance in the liquid to be measured; allowing an ion conductive compound moiety of the substance labeled with the ion conductive compound which is released from the immobilized antibody to be adsorbed onto a thin film which comprises a hydrocarbon group and is formed on a surface of one of electrodes ; and measuring a change in the electric conductivity of the thin film.

In one embodiment, the biological substance in the liquid to be measured is allowed to contact with the immobilized antibody to release, from the immobilized antibody, the substance which is labeled with the ion transmissible compound and binds to the immobilized antibody; an ion transmissible compound moiety of the released substance labeled with the ion transmissible compound is adsorbed onto the thin film formed on the surface of the working electrode; the amount of a change in the electric conductivity of the thin film that is induced by the adsorption of the ion conductive compound moiety is measured; and further the temperature of the liquid to be measured is optionally measured to determine the amount of the biological substance in the liquid to be measured.

In this embodiment, the biological substance and the substance labeled with the ion conductive compound each contain the site which the immobilized antibody recognizes and then binds to. In this case, the biological substance binds to the immobilized antibody while competing with the substance labeled with the ion conductive compound.

In this embodiment, in each of the biological substance and the substance labeled with the ion conductive compound, its site which the immobilized antibody recognizes may be entirely identical with an epitope of an antigen which can be used for preparing the immobilized antibody, or may be different therefrom as far as the immobilized antibody can bind to the site. For example, in the biological substance and the substance labeled with the ion conductive compound, respective amino acid sequences of their sites which the immobilized antibody recognizes may be identical with or different from each other as far as the immobilized antibody shows cross-reactivity (the difference is, for example, one residue, two residues, three residues, four residues or five residues).

In this embodiment, at least one of the biological substance and the substance labeled with the ion transmissible compound may be an antibody corresponding to the immobilized antibody. Moreover, the substance labeled with the ion conductive compound may be identical with the biological substance as a substance if the substance is not being labeled with the ion conductive compound, or different from the biological substance.

In one embodiment, the biological substance in the liquid to be measured is allowed to contact the substance labeled with the ion conductive compound and binds to the immobilized antibody, so as to release, from the immobilized antibody, the substance labeled with the ion transmissible compound; an ion transmissible compound moiety of the released substance labeled with the ion transmissible compound is adsorbed onto the thin film formed on the surface of the working electrode; the amount of a change in the electric conductivity of the thin film that is induced by the adsorption of the ion conductive compound moiety is measured; and further the temperature of the liquid to be measured is optionally measured to determine the amount of the biological substance in the liquid to be measured.

In this embodiment, the biological substance may be an antibody which recognizes the substance labeled with the ion conductive compound. In this way, the biological substance binds to the substance labeled with the ion conductive compound while competing with the immobilized antibody.

In this embodiment, the site of the substance labeled with the ion transmissible compound which the biological substance recognizes may be entirely identical with the recognition site of the substance labeled with the ion transmissible compound which the immobilized antibody recognizes or different from the recognition site as far as the substance labeled with the ion transmissible compound can bind to the sites. For example, in the site of the substance labeled with the ion transmissible compound which the biological substance recognizes, and the recognition site labeled with the ion transmissible compound which the immobilized antibody recognizes, respective amino acid sequences of these sites may be identical with or different from each other as far as the both antibodies bind thereto (the difference is, for example, one residue, two residues, three residues, four residues or five residues).

In this embodiment, the substance labeled with the ion conductive compound may be an antigen corresponding to at least one of the immobilized antibody and the biological substance. The immobilized antibody may be identical with the biological substance as a substance if the immobilized antibody is not immobilized or different from the biological substance.

In one embodiment, the immobilized antibody is a monovalent antibody.

In this embodiment, a reaction between the biological substance or the substance labeled with the ion conductive compound, and the immobilized antibody is limited only to a one-to-one reaction. Thus, the embodiment is excellent in quantitativity.

In one embodiment, the immobilized antibody is a monoclonal antibody.

In this embodiment, the affinity between the biological substance or the substance labeled with the ion conductive compound, and the immobilized antibody is uniform. Thus, the embodiment is excellent in quantitativity.

In one embodiment, the change amount of the electric conductivity is measured, using the application of an alternating voltage or the application of an alternating voltage to which a direct voltage bias is added.

In the case of only the application of a direct voltage, a current based on electrolysis (background) is involved, so that the SN ratio is not improved so much. In this embodiment, however, the current can be removed so that the SN ratio is remarkably improved.

In one embodiment, before the liquid to be measured contacts the substance labeled with the ion transmissible compound, the substance is binding to the immobilized antibody. In another embodiment, the substance labeled with the ion conductive compound is not binding to the immobilized antibody before the liquid to be measured contacts the substance. In this embodiment, the substance labeled with the ion transmissible compound is brought into contact with the immobilized antibody, simultaneously with the liquid to be measured or in the condition where the substance is beforehand mixed with the liquid to be measured.

Additionally, the method provided by the third aspect of the present invention can be performed with reference to the chip and the device provided by the first aspect of the invention.

D. Method for Measuring Amount of Biological substance in Liquid, Using Immobilized Substance and Antibody Labeled with Ion Conductive Material

It is described a method for measuring the amount of a biological substance in a liquid to be measured, comprising: allowing an antibody which is labeled with an ion conductive compound and binds to an immobilized substance to contact with the biological substance in the liquid to be measured; allowing an ion conductive compound moiety of the antibody labeled with the ion conductive compound which is released from the immobilized substance and to be adsorbed onto a thin film which comprises a hydrocarbon group and is formed on a surface of one of electrodes and; and measuring a change in the electric conductivity of the thin film.

In one embodiment, the biological substance in the liquid to be measured is allowed to contact with the immobilized substance to release, from the immobilized substance, the antibody which is labeled with the ion transmissible

compound and binds to the immobilized substance; an ion transmissible compound moiety of the released antibody labeled with the ion transmissible compound is adsorbed onto the thin film formed on the surface of the working electrode; the amount of a change in the electric conductivity of the thin film that is induced by the adsorption of the ion conductive compound moiety is measured; and further the temperature of the liquid to be measured is optionally measured to determine the amount of the biological substance in the liquid to be measured.

In this embodiment, the biological substance may be an antibody, and the antibody, which is the biological substance, and the antibody labeled with the ion conductive compound each recognize the immobilized substance to bind to this substance. In this case, the antibody, which is the biological substance, binds to the immobilized substance while competing with the antibody labeled with the ion conductive compound.

In this embodiment, sites where the antibody, which is the biological substance, and the antibody labeled with the ion conductive compound each recognize the immobilized substance may be entirely identical with or different from each other as far as the sites can bind to the immobilized substance. For example, in the antibody, which is the biological substance, and the antibody labeled with the ion conductive compound, respective amino acid sequences of their immobilized substance-recognizing sites may be identical with, or may be different from each other as far as the sites can bind to the immobilized substance (the difference is, for example, one residue, two residues, three residues, four residues or five residues).

In this embodiment, the immobilized substance may be an antigen corresponding to at least one of the biological substance and the antibody labeled with the ion conductive compound. The antibody labeled with the ion conductive compound may be identical with the biological substance as a substance if the antibody is not labeled with the ion conductive compound or different from the biological substance.

In one embodiment, the biological substance in the liquid to be measured is allowed to contact with the antibody which is labeled with the ion conductive compound and binds to the immobilized substance, so as to release, from the immobilized substance, the antibody labeled with the ion transmissible compound; an ion transmissible compound moiety of the released substance labeled with the ion transmissible compound is adsorbed onto the thin film formed on the surface of the working electrode; the amount of a change in the electric conductivity of the thin film that is induced by the adsorption of the ion conductive compound moiety is measured; and further the temperature of the liquid to be measured is optionally measured to determine the amount of the biological substance in the liquid to be measured.

In this embodiment, the biological substance may be a substance which binds to the antibody labeled with the ion conductive compound, and is, for example, a substance that may become an antigen corresponding to the antibody labeled with the ion conductive compound. In this way, the biological substance binds to the antibody labeled with the ion conductive compound while competing with the immobilized substance.

In this embodiment, the site of the biological substance which the antibody labeled with the ion transmissible compound recognizes may be entirely identical with the recognition site of the immobilized substance which the antibody labeled with the ion transmissible compound recognizes or different from the recognition site as far as the antibody labeled with the ion transmissible compound can bind to the sites. For example, in the site of the biological substance which the antibody labeled with the ion transmissible compound recognizes, and the recognition site of the immobilized substance which the antibody labeled with the ion transmissible compound recognizes, respective amino acid sequences of these sites may be identical with or different from each other as far as the both antibodies bind thereto (the difference is, for example, one residue, two residues, three residues, four residues or five residues).

In this embodiment, at least one of the immobilized substance and the biological substance may be an antigen corresponding to the antibody labeled with the ion transmissible compound. The immobilized substance may be identical with the biological substance as a substance if the immobilized substance is not immobilized or different from the biological substance.

In one embodiment, the immobilized antibody labeled with the ion transmissible compound is a monovalent antibody.

In this embodiment, a reaction between the antibody labeled with the ion transmissible compound, and the biological substance and the immobilized substance is limited only to a one-to-one reaction. Thus, the embodiment is excellent in quantitativity.

In one embodiment, the antibody labeled with the ion transmissible compound is a monoclonal antibody.

In this embodiment, the affinity of the antibody labeled with ion transmissible compound with the biological substance and the immobilized antibody is uniform. Thus, the embodiment is excellent in quantitativity.

In one embodiment, the change amount of the electric conductivity is measured, using the application of an alternating voltage or the application of an alternating voltage to which a direct voltage bias is added.

In the case of only the application of a direct voltage, a current based on electrolysis (background) is involved, so that the SN ratio is not improved so much. In this embodiment, however, the current can be removed so that the SN ratio is remarkably improved.

In one embodiment, before the liquid to be measured contacts the antibody labeled with the ion transmissible compound, this antibody is binding to the immobilized substance. In another embodiment, the antibody labeled with the

ion conductive compound is not binding to the immobilized substance before the liquid to be measured contacts the antibody. In this embodiment, the antibody labeled with the ion transmissible compound is brought into contact with the immobilized substance, simultaneously with the liquid to be measured or in the condition where the antibody is beforehand mixed with the liquid to be measured.

Additionally, the method provided by the fourth aspect of the present invention can be performed with reference to the chip and the device provided by the second aspect of the invention.

In one embodiment, the respective chips and devices provided by the first and second aspects of the present invention are usable to perform the respective methods provided by the third and fourth aspects of the invention.

E. Method and Device for Measuring Amount of Substance, Using Electrodes Containing Working Electrode Having, on Its Surface, Thin Film Containing Hydrocarbon Group, and Ion Conductive Compound

A fifth aspect of the present invention provides a device for measuring the concentration of a substance, comprising an ion conductive compound with which a substance is labeled for measuring the amount of the substance in a liquid to be measured; electrodes comprising a working electrode having a surface on which a thin film comprising a hydrocarbon group is formed, wherein the surface is a surface onto which the ion conductive compound is allowed to be adsorbed; an electric conductivity detecting section for detecting the electric conductivity of the thin film that is changed by allowing an ion conductive compound moiety of the substance labeled with the ion conductive compound to be adsorbed onto the working electrode; and a substance concentration calculating section for calculating the amount of the substance in the liquid to be measured based on a change in the electric conductivity.

A sixth aspect of the present invention provides a method for measuring the amount of a substance labeled with an ion conductive compound in a liquid to be measured, comprising: allowing an ion conductive compound moiety of the substance labeled with the ion conductive compound to be adsorbed onto a thin film which is formed on a surface of one of electrodes and comprises a hydrocarbon group; and measuring a change in the electric conductivity of the thin film.

In the fifth and sixth aspects, the electrodes may be composed a working electrode, which is an electrode on which an electrochemical reaction occurs, and a counter electrode, which is a reference electrode when a voltage is applied to between the electrodes. The electrodes may be made of a metal such as gold, platinum or silver, or a metal oxide such as ITO (indium tin oxide), $SnO_2$ ZnO, or $TiO_2$. The surface of the metal or metal oxide of the working electrode may be coated with a thin film containing a hydrocarbon group. The thin film may be a monomolecular film.

The thin film containing a hydrocarbon group is produced, for example, by reacting a metal with a hydrocarbon group having, at a terminal thereof, a group that can be bound to the metal, such as sulfide, diselenide, selenide, thiol, nitrile, isonitrile, nitro, selenol, a trivalent phosphorous compound, isothiocyanate, xanthate, thiocarbate, phosphine, thioacid, or dithioacid, to bind the group to the metal.

The hydrocarbon group is a linear fluoroalkyl group or a linear fluoroalkyl group containing a siloxane in each of which the alkyl group is a saturated or unsaturated alkyl group. The number of carbon atoms in each of these groups may be 1, 2, 3, 4, 5, 6, 7, 8, 9, 10, 11 or 12, and is, for example, 2, 3 or 4. Examples of these groups include $CF_3(CF_2)_{n-2}(CH_2)_2$- wherein n is 2, 3, 4, 5, 6, 7, 8, 9, 10 or 11, and $CF_3(CF_2)_{n-2}(CH_2)_2$-Si- wherein n is 2, 3, 4, 5, 6, 7, 8, 9, 10 or 11.

In one embodiment, a target substance, the amount of which is measured in the solution, is labeled with the ion conductive compound, and an ion conductive compound moiety thereof is allowed to be adsorbed onto one (the working electrode having, on its surface, the thin film) of the electrodes to form an ion channel, and the electric conductivity of the thin film that is changed through ions (such as sodium ions) in the solution is detected to measure the amount of the substance. Examples of the ion conductive compound include compounds each containing a crown ether group. An example thereof is as follows:

[Chem. 3]

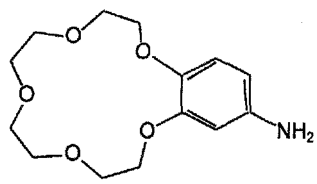

.

The system or method provided by the fifth and sixth aspects of the present invention can be performed with reference to the first to fourth aspects of the invention.

Hereinafter, exemplary embodiments of the present invention will be explained in detail with reference to the drawings. By attaching the same signs to the same members in the exemplary embodiments, an overlapping description

thereabout may be omitted in the following exemplary embodiments.

(Exemplary Embodiment 1)

In an exemplary method for measuring a biological substance provided by the present invention, a biological substance labeled with an ion conductive compound is adsorbed onto a thin film containing a hydrocarbon and formed on an electrode, and the biological substance labeled with the ion conductive compound is detected through the amount of a change in the electric conductivity of the thin film. This method can be performed through a mechanism as illustrated in Fig. 1.

A thin film 11 having a hydrocarbon group 12 and formed on an electrode is hydrophobic, and thus the film 11 acts onto an ion 1 as an electrically insulating film. However, when a biological substance 21 labeled with an ion conductive group 22 is adsorbed onto the thin film 11 having the hydrocarbon group 12 and formed on the electrode, the hydrocarbon group 12 of the thin film is oriented in a direction other than that of the ion conductive group since the hydrocarbon group 12 (hydrophobic group) of the thin film is different in affinity from the ion conductive group 22 (hydrophilic group). Thus, an ion channel 13 is formed from the ion conductive group 22 up to the upper of the electrode while penetrating through the thin film 11 having the hydrocarbon group 12. As a result, the ion 1 can move inside the thin film 11 so that the thin film 11 is changed in electric conductivity. Since the amount of the change corresponds to the adsorption amount of the biological substance 21 labeled with the ion conductive group 22 and adsorbed onto the thin film 11, the biological substance 21 labeled with the ion conductive group 22 and adsorbed onto the thin film 11 can be detected.

The raw material of the thin film 11 used for this embodiment may be a material to be bound to the electrode. When the electrode is, for example, a metal or metallic film, an R-SH based material can be used, where metal atom binds through the S atom to the hydrocarbon group 12. When the electrode is an oxide film, which may be a naturally metal-oxidized coat, an R-$SiX_3$ based material can be used, where a bond is made through the Si atom to the hydrocarbon group. A monomolecular film, on the surface of which the hydrocarbon group 12 is exposed, is formed by employing such structure according to Chemical Formula 1 or 2 to exhibit the performance of the present invention.

[Chem. 4]

(Chemical Formula 1)

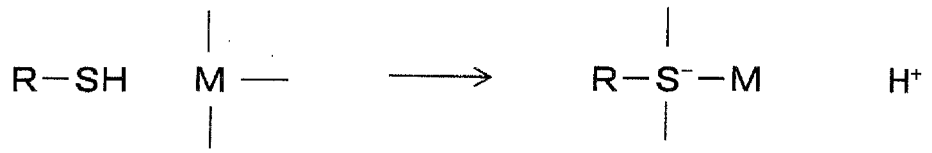

[Chem. 5]

(Chemical Formula 2)

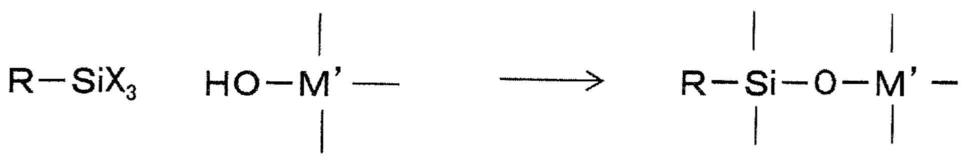

The hydrocarbon group may be a linear alkyl group or fluoroalkyl group. When the group is, in particular, a fluoroalkyl group, a more rigid molecular chain is produced so that a dense film can be formed. Thus, the SN ratio of the change amount of the electric conductivity becomes good.

If the hydrocarbon chain is short, the film becomes thin so that the blank value of the electric conductivity becomes large. On the other hand, if the chain is too long, the ion channel does not penetrate from the ion conductive group up to the surface of the electrode so that the change in the electric conductivity becomes small. Thus, in each of these cases, the SN ratio deteriorates. Accordingly, the hydrocarbon chain has an optimal length. In the case of, for example, a linear alkyl group or fluoroalkyl group, the number of carbon atoms therein may be from 3 to 12.

It is preferred that the hydrocarbon chain has no unsaturated bond. In this case, the film of the hydrocarbon chain becomes denser so that the SN ratio of the change of the electric conductivity becomes good. In view of these

matters, when the electrode is a metal, examples of the raw material of the thin film 11 include:

$CH_3(CH_2)_n$-SH wherein n is an integer of 2 to 11 (Chemical Formula 11), and
$CF_3(CF_2)_{n-2}(CH_2)_2$-SH wherein n is an integer of 2 to 11 (Chemical Formula 12).

When the electrode is an oxide film, which may be a naturally metal-oxidized coat, examples of the raw material include:

$CHs(CH_2)_n$-$SiX_3$ wherein n is an integer of 2 to 11 and X represents a halogen atom (Chemical Formula 21), and
$CF_3(CF_2)_{n-2}(CH_2)_2$-$SiX_3$ wherein n is an integer of 2 to 11 (Chemical Formula 22).

When the electrode is a metal or metallic film, the material of the metal may be a material from which a naturally oxidized coat is not easily formed, such as gold, platinum or silver. The use of a gold thin film makes the performance of the electrode compatible with costs.

When the electrode is an oxide or oxide film, the material of the oxide is, for example, ITO, doped $SnO_2$, ZnO or $TiO_2$, or any other transparent conductive film. The material may be a metal having a naturally oxidized coat. The material may be, for example, a metal having a naturally oxidized coat having a surface resistivity of 1 $k\Omega/cm^2$ or less.

The ion conductive compound may be a compound having an ionophore that forms an ion channel, such as an acetylcholine receptor in a neural synapse. Examples thereof include valinomycin and nigericin as natural products. When these can each be modified into an ion conductive group, these are usable. The ion conductive group that is a synthesized product may be a group having a cyclic structure where an orientation structure has large inward polarity and small outward polarity. The outward moiety of the hydrophobic cyclic structure attracts the hydrophobic hydrocarbon group through hydrophobic bonding, whereby the ion conductive group penetrates through the hydrocarbon group-containing thin film so that a channel is formed up to the upper of the electrode, and further ions are easily introduced into the region of the channel by the inner moiety of the cyclic structure. As a result, based on a change in the electric conductivity of the thin film, the biological substance labeled with the ion conductive group can be detected. Such a structure may be a crown ether structure represented by 22 in Fig. 1. The outer moiety of the ring of the crown ether attracts the hydrocarbon chain so that an ion channel is formed in the thin film. From the inner moiety of the ring of the crown ether, for example, sodium ions contained in a biological liquid are introduced into the ion channel to cause a change in the electric conductivity, thereby making it possible to detect the biological substance labeled with the ion conductive group.

Thus, the crown ether is an ether through which sodium ions can permeate. The structure of the ether is preferably an aminobenzyl-15-crown-5 residue represented by 22 in Fig. 1, or a cyclic structure having a size equal to or more than that of the residue. The crown ether may be a crown ether containing -NH-radicals or -S-radials instead of the O-radicals.

(Exemplary Embodiment 2)

An exemplary method for measuring a biological substance (antigen) provided by the present invention has an immobilized antibody and a labeled antigen which is labeled with an ion conductive compound and binds to the immobilized antibody by an antigen-antibody reaction. In the method, the immobilized antibody and the labeled antigen are allowed to contact with a biological substance (antigen) in a liquid to be measured to release the labeled antigen by a replacement reaction between the biological substance (antigen) and the labeled antigen; the released labeled antigen is adsorbed onto a thin film which is formed on a surface of an electrode and contains a hydrocarbon group; and the labeled antigen labeled with the ion conductive compound is detected based on a change in the electric conductivity of the thin film. This method can be performed through a mechanism as illustrated in Fig. 2.

A biological substance (antigen) 120 is brought into contact with a labeled antigen 121 which is labeled with an ion conductive group 122 and binds onto an immobilized antibody 130 by an antigen-antibody reaction (Complex of labeled antigen and immobilized antibody 131) (the labeled antigen 121 has an antigen region 123 having a binding affinity to the immobilized antibody 130, and the affinity is equivalent to the affinity of the biological substance (antigen) 120). As a result, the labeled antigen 121 labeled with the ion conductive group, which is large in molecular weight, is replaced with the biological substance (antigen) 120, so that the labeled antigen 121 is released. When this released antigen 121 labeled with the ion conductive group 122 is adsorbed onto a thin film 111 formed on a surface of an electrode and having a hydrocarbon group 112, the hydrocarbon group 112 of the thin film is oriented in a direction other than that of the ion conductive group since the hydrocarbon group 112 (hydrophobic group) of the thin film is different in affinity from the ion conductive group 122 (hydrophilic group). For this reason, an ion channel 113 is formed from the ion conductive group 122 up to the upper of the electrode while penetrating through the thin film 111 having the hydrocarbon group 112. As a result, an ion 101 can move inside the thin film 111 to change the electric conductivity of the thin film

111. The amount of the change corresponds to the amount of adsorption of the antigen 121 labeled with the ion conductive group 122 and adsorbed onto the thin film 111, so that the antigen 121 labeled with the ion conductive group 122 and adsorbed onto the thin film 111 can be detected. In short, the replaced biological substance (antigen) 120 can be detected.

The raw material of the thin film 11, the electrode, and the ion conductive group that are used at this time are as described in the exemplary embodiment 1. Thus, any detailed description thereof is omitted.

The antigen 120 used at this time is a substance that binds to an antigen receptor on an immune cell to induce an immune reaction. An example thereof is an ovarian hormone, estradiol, 120A. In Fig. 3 is shown a labeled antigen 121A in which a raw material 122A of the ion conductive group 122 is introduced into this hormone.

As illustrated in Fig. 4, the immobilized antibody 131 used at this time is synthesized, as an example 131A thereof, as follows.

The antibody 130 is generally a tetramer in which four protein chains of two long H chains 130H and two short L chains 130L are bound each other through disulfide bonds 130S. The figure schematically illustrates this structure. The parallel chain moieties in each of which the H chain 130H binds to the L chain 130L through the disulfide bond 130S each further exhibit a spatial configuration to attain a specific recognition and binding to, for example, the labeled antigen 121A through an antigen-antibody reaction. Accordingly, the antibody is necessarily bivalent to be capable of binding to two antigen molecules.

Next, polystyrene beads 132A selected as an example of an immobilizing substrate 132 have a molecular structure in which side chains of phenyl groups bind to the main chain of a hydrocarbon. When a bromine molecule is allowed to act onto this polystyrene molecule, a brominated polystyrene molecule 132B is obtained in which hydrogen atoms of the phenyl groups are substituted with bromine atoms. The brominated polystyrene is active to react with a protein such as an antibody, so that crosslinking bonds using the phenyl groups can be formed. In this way, the immobilized antibody 131A is obtained.

Besides the illustrated method, many methods for the immobilization are known. As the shape of the substrate, besides the shape of the beads, many shapes are known. The method for the immobilization is not limited to the illustrated method.

The bivalent antibody 130 should have, in light of the symmetry thereof, equivalent binding affinity to two molecules of the antigen 120 that are identical with each other. However, when one of the moieties of the antibody binds to one of the molecules of the antigen 120, the other moiety does not bind easily to the other molecule by obstruction (steric hindrance) based on the already bound antigen. In short, the first one of the antigen molecules is different in binding affinity from the second one. In the present embodiment, description has been made about the replacement reaction between the labeled antigen on the immobilized antibody and the antigen which is a substance to be measured. However, as far as a bivalent antibody is used, the first and second antigen molecules are not equal to each other in replacement probability. Thus, change of the concentration does not become linear so that an accidental error in the electric conductivity change is caused. This is overcome by the use of a monovalent antibody.

As illustrated in Fig. 5, a monovalent antibody 135 can be obtained by allowing a protease, such as porcine pepsin, to act onto the bivalent antibody 130 so as to cleave H chain portions not concerned with the antigen-antibody reaction, and further allowing a reducing agent such as 2-mercaptoethylamine to act onto the partially-cleaved antibody to cleave the disulfide bonds. In the thus obtained monovalent antibody 135, its thiol groups, which are not involved with the antigen-antibody reaction, are exposed. A method of crosslinking these thiol groups to each other with a bivalent crosslinking agent to yield an immobilized antibody is called as the hinge method. The method is useful as an immobilizing method in which the potency of an antibody is not decreased, for the present invention.

By immunizing an antigen to an experimental animal such as a rat, mouse, rabbit, goat or bovine, many kinds of antibodies corresponding to the antigen are produced in its blood plasma. Inside the body of the experimental animal, antibody-producing immune cells produce one antibody per one cell. Antibody diversity can be generated by the production of different antibodies against the same antigen by many immune cells. Such an antibody is called as a polyclonal antibody. By contrast, a cell obtained by taking out only one immune cell and making the immune cell immortal (juvenile) by cell fusion with a myeloma cell produces only one kind of antibody. This antibody is called as a monoclonal antibody.

A polyclonal antibody is a mixture of many antibodies that recognize different recognition sites of the same antigen to bind to the sites. Thus, between the antibodies, their respective binding affinities to the antigen are different from each other. The composition thereof is varied in accordance with the production conditions, and is not constant. When a polyclonal antibody is used as an antibody, worse linearity is generally found between the concentration of antigen in a substance to be measured and measured results (the electric conductivity), and an accidental error easily arises, thereby being worse in reproducibility than the case of using a monoclonal antibody. By contrast, when a monoclonal antibody, which is a uniform antibody, is used, good linearity is found between the concentration of antigen in a substance to be measured and measured results (the electric conductivity), and an accidental error is difficult to arise, thereby being good in reproducibility.

(Exemplary Embodiment 3)

An exemplary method for measuring an antigen provided by the present invention has an immobilized antigen, and a labeled antibody labeled with an ion conductive group and binding to the immobilized antigen by an antigen-antibody reaction. In the method, the immobilized antigen and the labeled antibody are brought into contact with an antibody in a liquid to be measured to release the labeled antigen by a replacement reaction between the antigen and the labeled antigen; the released labeled antigen is adsorbed onto a thin film formed on a surface of an electrode and containing a hydrocarbon group; and the labeled antibody labeled with the ion conductive group is detected based on a change in the electric conductivity of the thin film. This method can be performed through a mechanism as illustrated in Fig. 6.

An antibody 230 is brought into contact with a labeled antibody 231 which is labeled with an ion conductive group 232 and binds onto an immobilized antigen 220 by an antigen-antibody reaction (Complex of labeled antibody and immobilized antigen) (the labeled antibody 231 has an antibody region 233 having a binding affinity to the immobilized antigen 220, and the affinity is equivalent to the affinity of the antibody 230). As a result, the labeled antibody 231 labeled with the ion conductive group, which is large in molecular weight, is replaced with the antibody 230, so that the labeled antibody 231 is released. When the released antibody 231 labeled with the ion conductive group 232 is adsorbed onto a thin film 211 formed on a surface of an electrode and having a hydrocarbon group 212, the hydrocarbon group 212 of the thin film is oriented in a direction other than that of the ion conductive group since the hydrocarbon group 212 (hydrophobic group) of the thin film is different in affinity from the ion conductive group 232 (hydrophilic group). For this reason, an ion channel 213 is formed from the ion conductive group 232 up to the upper of the electrode while penetrating through the thin film 211 having the hydrocarbon group 212. As a result, an ion 201 can move inside the thin film 211 to change the electric conductivity of the thin film 211. The amount of the change corresponds to the amount of adsorption of the antigen 231 labeled with the ion conductive group 232 and adsorbed onto the thin film 211, so that the antigen 231 labeled with the ion conductive group 232 and adsorbed onto the thin film 211 can be detected. In short, the replaced antigen 230 can be detected.

The raw material of the thin film 11, the electrode, and the ion conductive group that are used at this time are as described in the exemplary embodiment 1. Thus, any detailed description thereof is omitted.

As illustrated in Fig. 7, the immobilized antigen 221 used at this time is synthesized, as an example 221A thereof, as follows.

The antigen 220 is a substance that binds to an antigen receptor on an immune cell to cause an immune reaction. An example thereof is an ovarian hormone, estradiol, 220A.

Next, polystyrene beads 222A selected as an example of a substrate 222 have a molecular structure in which side chains of phenyl groups bind to the main chain of a hydrocarbon. When an amino group is introduced into the polystyrene, an aminated polystyrene molecule 221B is obtained.

The aminated polystyrene is bound through a crosslinking agent to estradiol to obtain the immobilized antigen 221A.

In another method for producing the immobilized antigen, a bromine molecule is allowed to act onto polystyrene to prepare a brominated polystyrene molecule in which hydrogen atoms of phenyl groups are substituted with bromine atoms, and then the brominated polystyrene is subject to reaction with a protein such as an antibody to form crosslinking bonds using the phenyl groups. In this way, the immobilized antigen is obtained.

Besides the illustrated method, many methods for the immobilization are known. As the shape of the substrate, besides the shape of the beads, many shapes are known. The method for the immobilization is not limited to the illustrated method.

The antibody 230 is generally a tetramer in which four protein chains of two long H chains 230H and two short L chains 230L are bound each other through disulfide bonds 230S. The figure schematically illustrates this structure thereof. The parallel chain moieties in each of which the H chain 230H binds to the L chain 230L through the disulfide bond 230S each further exhibit a spatial configuration to attain a specific recognition and binding to, for example, the immobilized antigen 220A through an antigen-antibody reaction. Accordingly, the antibody is necessarily bivalent to be capable of binding to two antigen molecules. In Fig. 8 is shown a labeled antibody 231A in which a raw material 232A of the ion conductive group 232 is introduced into this antibody.

The monovalent antibody and monoclonal antibody each used at this time are as explained in the exemplary embodiment 2. Thus, any detailed description thereof is omitted.

(Exemplary Embodiment 4)

An exemplary device for measuring an antigen provided by the present invention comprises an antigen-measuring chip having an immobilized antibody; a labeled antigen which is labeled with an ion conductive substance and binds to the immobilized antibody by an antigen-antibody reaction; a thin film which is formed on a surface of an electrode

and contains a hydrocarbon group; a counter electrode, and a section for measuring the temperature of a liquid to be measured, where the chip is brought into contact with an antigen in the liquid to be measured to release the labeled antigen by a replacement reaction between the antigen and the labeled antigen; the released labeled antigen is absorbed onto the thin film which is formed on the electrode surface and contains the hydrocarbon group to measure a change in the electric conductivity of the thin film and the temperature of the liquid to be measured. The present device also comprises an operating device that is connected to the electrode and the counter electrode of the measuring chip, and further to a section for measuring the temperature of the electrode, and that detects the ion conductive labeled antigen based on a change in the electric conductivity of the thin film and a correction of the temperature, thereby measuring the antigen. The device can be attained by configuration in Fig. 9. In this embodiment, in a case where the temperature at the time of measuring the antigen is constant (the temperature is, for example, a constant temperature of 25°C), the temperature correction may not be made. Among the constituents shown in Fig. 9, a section 359 for measuring the temperature of the liquid to be measured, and a section 362 for detecting the electrode temperature may not exist. Examples of the case where the temperature is constant include a case where the liquid to be measured is collected from a living body and the temperature thereof is sufficiently returned to room temperature, and subsequently the measuring device of the present embodiment is used to measure the liquid; and a case where the liquid to be measured is collected from a living body at room temperature and immediately used as it is, and the temperature of the liquid to be measured is a constant temperature of about 37°C while the measuring device of the present embodiment is used to measure this liquid.

In a measuring chip 350, an electrode film for a working electrode 352 is formed on a substrate 351, and a pipe wall 353 is formed thereon to form a liquid-supplying section 354 and a capillary liquid-absorbing section 355. Furthermore, a thin film 112 containing a hydrocarbon group is formed on the electrode film for the working electrode 352. Additionally, electric wire sections 356 are formed, and then a complex of labeled antigen and immobilized antibody 131 is filled into a labeled antigen and immobilized antibody filling section 357 so as to be filled up to the capillary liquid absorbing section 355. Thereafter, so as not to be filled into the capillary liquid absorbing section 355, a counter electrode 358 is set and, when the temperature of the liquid to be measured is measured, the liquid temperature measuring section 359 is set. Finally, a film is laminated to cover at least the capillary liquid absorbing section 355, the working electrode 352, the counter electrode 358, and the immobilized antibody section 357, and when the temperature of the liquid to be measured is measured, cover the liquid temperature measuring section 359 so as not to be filled into the capillary liquid absorbing section 355. In this way, the measuring chip 350 is produced.

The following will describe the action of the device. When a liquid to be measured that contains an antigen 120 is supplied into the liquid supplying section 354 of the measuring chip 350, the liquid to be measured is taken into the capillary liquid-absorbing section 355 by a capillary phenomenon. When the liquid to be measured reaches the immobilized antibody filling section 357, the antigen 120 in the liquid to be measured replaces a labeled antigen 121 which is labeled with an ion conductive group 122 and binds onto an immobilized antibody 130 by an antigen-antibody reaction so that the labeled antigen 121 is released. When the liquid to be measured is further taken into the capillary liquid absorbing section 355 by a capillary phenomenon in a case where the temperature of the liquid to be measured should be measured, the liquid temperature of the liquid to be measured is measured in the liquid temperature measuring section 359 and the temperature is detected in the temperature detecting section 362 inside the operating device. Furthermore, when the released labeled antigen 121 is adsorbed onto the thin film 111 having a hydrocarbon group 112 and formed on the electrode, the hydrocarbon group 112 of the thin film is, on the working electrode 352, oriented in a direction other than that of the ion conductive group since the hydrocarbon group 112 (hydrophobic group) of the thin film is different in affinity from the ion conductive group 122 (hydrophilic group). Thus, an ion channel 113 is formed from the ion conductive group 122 up to the upper of the electrode while penetrating through the thin film 111 having the hydrocarbon group 112. As a result, an ion 101 can move inside the thin film 111 to cause a change in the electric conductivity of the working electrode 352 relative to that of the counter electrode 358. The change is then detected in the electric conductivity detecting section 361 inside the operating device. This amount of change of the electric conductivity corresponds to the amount of adsorption of the antibody 121 labeled with the ion conductive group 122 and adsorbed onto the thin film 111. Thus, in the antigen concentration calculating section 363, the antigen 120 can be precisely detected while making a correction according to data in the temperature detecting section 362 if necessary.

(Exemplary Embodiment 5)

An exemplary device for measuring an antibody provided by the present invention has an antibody-measuring chip having an immobilized antigen; a labeled antibody which is labeled with an ion conductive substance and binds to the immobilized antigen by an antigen-antibody reaction; a thin film which is formed on a surface of an electrode and contains a hydrocarbon group; a counter electrode; and a section for detecting the temperature of the electrode; where the chip is brought into contact with an antibody in a liquid to be measured to release the labeled antibody by a replacement reaction between the antibody and the labeled antibody; the released labeled antibody is absorbed onto the thin film

which is formed on the electrode surface and contains the hydrocarbon group to measure a change in the electric conductivity of the thin film and the temperature. The electrode and the counter electrode of the measuring chip are connected to the section for detecting the temperature of the electrode to detect the ion conductive labeled antibody based on a change in the electric conductivity of the thin film, and a correction of the temperature. This device can be attained by a mechanism in Fig. 10. In this embodiment, in a case where the temperature at the time of measuring the antibody is constant (the temperature is, for example, a constant temperature of 25°C), the temperature correction may not be made. Among the constituents shown in Fig. 10, a section 459 for measuring the temperature of the liquid to be measured, and a section 462 for detecting the temperature of the electrode may not exist. Examples of the case where the temperature is constant include a case where the liquid to be measured is collected from a living body and the temperature thereof is sufficiently returned to room temperature, and subsequently the measuring device of the present embodiment is used to measure the liquid; and a case where the liquid to be measured is collected from a living body at room temperature and immediately used as it is, and the temperature of the liquid to be measured is a constant temperature of about 37°C while the measuring device of the present embodiment is used to measure this liquid.

In a measuring chip 450, an electrode film for a working electrode 452 is formed on a substrate 451, and a pipe wall 453 is formed thereon to form a liquid supplying section 454 and a capillary liquid absorbing section 455. Furthermore, a thin film 212 containing a hydrocarbon group is formed on the electrode film for the working electrode 452. Additionally, electric wire sections 456 are formed, and then a complex of labeled antibody and immobilized antigen 221 is filled into a complex of labeled antibody and immobilized antigen filling section 457 so as to be filled up to the capillary liquid absorbing section 455. Thereafter, so as not to be filled into the capillary liquid- absorbing section 455, a counter electrode 458 is set and, when the temperature of the liquid to be measured is measured, the liquid temperature measuring section 459 is set. Finally, a film is laminated to cover at least the capillary liquid absorbing section 455, the working electrode 452, the counter electrode 458, and the immobilized antigen section 457, and when the temperature of the liquid to be measured is measured, cover the liquid temperature measuring section 459 so as not to be filled into the capillary liquid absorbing section 455. In this way, the measuring chip 450 is produced.

The following will describe the action of the device. When a liquid to be measured that contains an antibody 230 is supplied into the liquid supplying section 454 of the measuring chip 450, the liquid to be measured is taken into the capillary liquid-absorbing section 455 by a capillary phenomenon. When the liquid to be measured reaches the immobilized antigen filling section 457, the antibody 230 in the liquid to be measured replaces a labeled antibody 231 which is labeled with an ion conductive group 232 and binds onto an immobilized antigen 220 by an antigen-antibody reaction so that the labeled antibody 231 is released. When the liquid to be measured is further taken into the capillary liquid absorbing section 455 by a capillary phenomenon in a case where the temperature of the liquid to be measured should be measured, the liquid temperature of the liquid to be measured is measured in the liquid temperature measuring section 459 and the temperature is detected in the temperature detecting section 462 inside the operating device. Furthermore, when the released labeled antibody 231 is adsorbed onto the thin film 211 having a hydrocarbon group 212 and formed on the electrode, the hydrocarbon group 212 of the thin film is, on the working electrode 452, oriented in a direction other than that of the ion conductive group since the hydrocarbon group 212 (hydrophobic group) of the thin film is different in affinity from the ion conductive group 232 (hydrophilic group). Thus, an ion channel 213 is formed from the ion conductive group 232 up to the upper of the electrode while penetrating through the thin film 211 having the hydrocarbon group 212. As a result, an ion 201 can move inside the thin film 211 to cause a change in the electric conductivity of the working electrode 452 relative to that of the counter electrode 457. The change is then detected in the electric conductivity detecting section 461 inside the operating device. This amount of change of the electric conductivity corresponds to the amount of adsorption of the antibody 231 labeled with the ion conductive group 232 and adsorbed onto the thin film 211. Thus, in the antigen concentration calculating section 463, the antibody 230 can be precisely detected while making a correction according to data in the temperature detecting section 462 if necessary.

Additional non-limiting embodiments provided by the present invention include the following (1) to (18).

(1) A chip for measuring the amount of a biological substance present in a liquid to be measured, comprising

an immobilized antibody; a substance which is labeled with an ion conductive compound and binds to the immobilized antibody; and electrodes comprising a working electrode and a counter electrode, wherein
the working electrode has, on its surface, a thin film comprising a hydrocarbon group.

(2) A chip for measuring the amount of a biological substance present in a liquid to be measured, comprising

a chip comprising an immobilized substance; an antibody which is labeled with an ion conductive compound and binds to the immobilized substance; and electrodes comprising a working electrode and a counter electrode, wherein
the working electrode has, on its surface, a thin film comprising a hydrocarbon group.

(3) The chip according to (1) or (2), further comprising a section for measuring the temperature of the liquid to be measured.

(4) A device for measuring a biological substance in a liquid to be measured, comprising the chip recited in any one of (1) to (3).

(5) A method for measuring the amount of a biological substance in a liquid to be measured, comprising: allowing an immobilized antibody which binds to a substance labeled with an ion conductive compound to contact with the biological substance in the liquid to be measured; allowing an ion conductive compound moiety of the substance labeled with the ion conductive compound which is released by a replacement reaction between the biological substance and the substance labeled with the ion conductive compound to be adsorbed onto a thin film formed on a surface of an electrode and containing a hydrocarbon group; and measuring a change in the electric conductivity of the thin film.

(6) A method for measuring the amount of a biological substance in a liquid to be measured, comprising: allowing an antibody which is labeled with an ion conductive compound and binds to an immobilized substance to contact with the biological substance in the liquid to be measured; allowing an ion conductive compound moiety of the antibody labeled with the ion conductive compound which is released by a replacement reaction between the biological substance and the antibody labeled with the ion conductive compound to be adsorbed onto a thin film formed on a surface of an electrode and containing a hydrocarbon group; and measuring a change in the electric conductivity of the thin film.

(7) The chip according to (1) or (3), the device according to (4) or the method according to (5), wherein the substance labeled with the ion conductive compound is released from the immobilized antibody due to the presence of the biological substance in the liquid to be measured;

an ion conductive compound moiety of the released substance labeled with the ion conductive compound is adsorbed onto the thin film formed on the surface of the working electrode; and

the amount of a change in the electric conductivity of the thin film that is induced by the adsorption of the ion conductive compound moiety is measured, and optionally the temperature is measured, thereby determining the amount of the biological substance in the liquid to be measured.

(8) The chip according to (2) or (3), the device according to (4) or the method according to (6), wherein the antibody labeled with the ion conductive compound is released from the immobilized substance due to the presence of the biological substance in the liquid to be measured;

an ion conductive compound moiety of the released antibody labeled with the ion conductive compound is adsorbed onto the thin film formed on the surface of the working electrode; and

the amount of a change in the electric conductivity of the thin film that is induced by the adsorption of the ion conductive compound moiety is measured, and optionally the temperature is measured, thereby determining the amount of the biological substance in the liquid to be measured.

(9) The chip, the device or the method according to any one of (1) to (8), wherein the immobilized antibody or the antibody labeled with the ion conductive compound is a monovalent antibody.

(10) The chip, the device or the method according to (9), wherein the immobilized antibody or the antibody labeled with the ion conductive compound is a monoclonal antibody.

(11) A device for measuring the concentration of a substance, comprising an ion conductive compound with which a substance is labeled for measuring the amount of the substance (for example, a biological substance) in a liquid to be measured; electrodes comprising a working electrode having a surface on which a thin film containing a hydrocarbon group is formed, wherein the thin film-formed surface is a surface onto which the ion conductive compound is allowed to be adsorbed; an electric conductivity detecting section for detecting the electric conductivity of the thin film that is changed by allowing an ion conductive compound moiety of the substance labeled with the ion conductive compound to be adsorbed onto the working electrode; and a substance concentration calculating section for calculating the amount of the substance in the liquid to be measured based on a change in the electric conductivity.

(12) A method for measuring the amount of a substance (for example, a biological substance) labeled with an ion conductive compound in a liquid to be measured, comprising: allowing an ion conductive compound moiety of the substance labeled with the ion conductive compound to be adsorbed onto a thin film formed on a surface of an electrode and containing a hydrocarbon group; and measuring a change in the electric conductivity of the thin film.

(13) The chip, the device or the method according to any one of (1) to (12), wherein the hydrocarbon group is a linear alkyl group, a linear fluoroalkyl group, a linear alkyl group containing a siloxane, or a linear fluoroalkyl group containing a siloxane, and the number of carbon atoms in each of these groups is preferably 1, 2, 3, 4, 5, 6, 7, 8,

9, 10, 11 or 12 (for example, $CH_3(CH_2)_n$- wherein n is 1, 2, 3, 4, 5, 6, 7, 8, 9, 10 or 11, $CF_3(CF_2)_{n-2}(CH_2)_2$-wherein n is 1, 2, 3, 4, 5, 6, 7, 8, 9, 10 or 11, $CH_3(CH_2)_n$-Si- wherein n is 1, 2, 3, 4, 5, 6, 7, 8, 9, 10 or 11, or $CF_3(CF_2)_{n-2}(CH_2)_2$-Si- wherein n is 1, 2, 3, 4, 5, 6, 7, 8, 9, 10 or 11).

(14) The chip, the device or the method according to (13), wherein the number of the carbon atoms is from 1 to 8, preferably 2, 3, or 4, more preferably 4.

(15) The chip, the device or the method according to any one of (1) to (14), wherein the thin film is a monomolecular film.

(16) The chip, the device or the method according to any one of (1) to (15), wherein the ion conductive compound comprises an ion conductive group, and the ion conductive group is a group having a cyclic structure which has an orientation structure having large inward polarity and small outward polarity (for example, a group having a crown ether structure (for example, a structure represented by $(-CH_2-CH_2-O-)_n$ wherein n is 5 or more)).

(17) The chip, the device or the method according to any one of (1) to (16), wherein the ion conductive compound comprises an ion conductive group, and the cyclic structure of the ion conductive group has a size equal to or more than such a size that a sodium ion can undergo clathration.

(18) The chip, the device or the method according to any one of (1) to (17), wherein the ion conductive compound is a compound containing an aminobenzyl-15-crown-5 residue (for example, estradiol having a 15-crown-5 residue (for example, the compound of the labeled antigen 121A in Fig. 3)).

(19) The chip, the device or the method according to any one of (1) to (18), wherein the amount of the change in the electric conductivity is measured through the application of an alternating voltage or the application of an alternating voltage to which a direct voltage bias is added.

(20) The chip, the device or the method according to any one of (1) to (19), wherein the biological substance is estradiol.

(21) The chip, the device or the method according to any one of (2) to (4), (6) to (10), and (13) to (20), wherein the immobilized substance is estradiol binding through a crosslinking agent to a support containing polystyrene (for example, estradiol immobilized onto polystyrene beads, using, as the crosslinking agent, disuccinyldisulfide as illustrated in Fig. 7).

Hereinafter, examples of the present invention will be explained in detail with reference to the drawings.

Examples

(Example 1)

A measuring chip for a device for measuring an antibody, as illustrated in Fig. 9, in the exemplary embodiment 4 provided by the present invention was produced by the following methods.

A gold electrode film, 100 nm in thickness, is formed as a working electrode 352 onto a glass substrate 351, and an epoxy resin is printed thereon and cured to form a pipe wall 353 having a thickness of 50 $\mu$m, thereby forming a liquid supplying section 354 and a capillary liquid absorbing section 355 having a cross section 2 mm x 50 $\mu$m in size and having a contacting length of 10 mm between the working electrode 352 and a liquid. Furthermore, a 1% by volume $C_4H_9SH$ solution in $CH_3CH_2OH$ is coated onto the electrode film for the working electrode 351, and dried to form a thin film 112 having a hydrocarbon group $C_4H_9$ as shown by Chemical Formula 1. Furthermore, a conductive paste is printed thereon and dried to form electric wire sections 356, and then a complex of labeled estradiol antibody and polystyrene bead immobilized antibody 131A is filled into an immobilized antibody filling section 357 to be filled up to the capillary liquid absorbing section 355. Thereafter, so as not to be filled into the capillary liquid absorbing section 355, a graphite sheet is set as a counter electrode 358 to have substantially the same area as the working electrode, and a temperature IC chip is formed as a liquid temperature measuring section 359. Finally, an epoxy ultraviolet resin is coated to cover at least the capillary liquid absorbing section 355, the working electrode 352, the counter electrode 358, and the immobilized antibody section 357 and the liquid temperature measuring section 359, so as not to be filled into the capillary liquid absorbing section 355. The resin is then photo-cured. In this way, a measuring chip 350 is produced.

An electric conductivity meter was connected to the electric wire section to which the working electrode 352 and the counter electrode 358 of this chip were connected, and a temperature displaying circuit for temperature IC chip was connected to the electric wire section to which a temperature IC chip of the liquid temperature measuring section 359 was connected. A liquid to be measured containing 40 to 400000 ng/mL of an estradiol antigen was supplied into the liquid supplying section 354, and an alternating voltage of 0.6 V, the frequency of which was 60 Hz, was applied to the chip 350, and the electric conductivity thereof was measured. The results are shown in Fig. 11. The temperature of the liquid to be measured was 25°C at this time. Thus, the display of the temperature displaying circuit for temperature IC chip was corrected to be turned to 25°C.

(Relationship between Concentration and Electric Conductivity)

Fig. 11 shows a change in the electrode relative to the concentration C of the estradiol antigen. By an antigen-antibody reaction, the antigen is completely replaced with a labeled antigen so that the concentration of the labeled antigen turns to C. It is considered that the electric conductivity $\sigma$ at this time is in proportion to the coverage ratio $\theta$ of the absorbed labeled antigen. Thus,

$$\sigma = K1 \times \theta \qquad \text{(Equation 1)}$$

wherein K1: response constant.

When it is supposed that this adsorption obeys Langmuir isothermal adsorption, the following is satisfied at the time of desorption equilibrium (desorption rate = adsorption rate):

$$kd \times \theta S = ka \times (1 - \theta)S \times C \qquad \text{(Equation 2)}$$

wherein $\theta$: the coverage ratio of the adsorbed labeled antigen,
S: the adsorption area,
C: the concentration of the released labeled antigen,
kd: desorption rate constant, and
ka: adsorption rate constant.

Thus, the coverage ratio $\theta$ of the adsorbed labeled antigen satisfies:

$$\theta = 1/(1 + K2/C) \qquad \text{(Equation 3),}$$

and

$$K2 = Kd/Ka \qquad \text{(Equation 4)}$$

wherein K2: desorption equilibrium constant.

Thus, from Equations 1 and 2, the electric conductivity satisfies:

$$\sigma = K1 \times \theta = K1/(1 + K2/C) \qquad \text{(Equation 5)}$$

The reciprocal number of this value is:

$$1/\sigma = 1/K1 + (K2/K1)C \qquad \text{(Equation 6)}$$

It is understood that when $1/\sigma$ and 1/C are used to convert Fig. 11, a linear relationship as shown in Fig. 12 is obtained. Consequently, it was verified that the labeled antigen undergoes Langmuir isothermal adsorption in accordance with the concentration thereof, and thus ion channels are formed so that ions pass through the film, whereby a change in the electric conductivity, that is, an electric response is made.

(Relationship between Temperature and Electric Conductivity)

Next, a liquid to be measured containing 800 ng/mL of an estradiol antigen was supplied at 25 to 45°C, and then the electric conductivity was measured. The results are shown in Fig. 13. From Equation 5, the following is obtained:

$$K2 = (K1/\sigma - 1)/C \qquad \text{(Equation 7)}$$

K2 has the following relationship with absolute temperature T:

$$\ln K2 = -\Delta G/RT \qquad \text{(Equation 8)}$$

wherein $\Delta G$: a change in the free energy of the desorption equilibrium reaction.

Thus, from Equations 7 and 8, the following is obtained:

$$\ln(1/\sigma) = -\Delta G/RT + K3 \qquad \text{(Equation 9)}$$

$$K3 = \ln C - \ln K1 \qquad \text{(Equation 10)}$$

It is understood that when ln(1/σ) and 1/T are used to convert Fig. 13, a linear relationship shown in Fig. 14 is obtained.

According to the above, by supplying liquids to be measured that have an unknown estradiol concentration and have various temperatures, the concentration of estradiol is measurable.

(Comparative Example 1)

Next, a phospholipid bilayer membrane was formed by coating a phosphatidylcholine solution in toluene, and then drying the coated solution instead of the formation of the thin film 112 having the hydrocarbon group $C_4H_9$ as shown by Chemical Formula 1 by coating and drying the 1vol % of $C_4H_9SH$ solution in $CH_3CH_2OH$ on the electrode film for the working electrode 351 as in Example 1. In the same way as in Example 1, a liquid to be measured containing an estradiol antigen (mol/L) was supplied thereto, and the electric conductivity was measured. The results are shown in Fig. 15. It is understood that when 1/σ and 1/C are used to convert Fig. 15, a linear relationship as shown in Fig. 16 is obtained. Consequently, it was verified that the labeled antigen undergoes Langmuir isothermal adsorption in accordance with the concentration thereof, and thus ion channels are formed so that ions pass through the membrane, whereby a change in the electric conductivity, that is, an electric response is made. However, according to comparison between the resultant linear relationship equation and Equation 6, the response constant K1 in Example 1 is 2.45E-05(S), and the response constant K1 in Comparative Example 1 is 4.93E-08(S) to be three digits smaller than the former.

When Equation 5 is differentiated, the following is obtained:

$$d\sigma/dc = K1/K2 \qquad \text{(Equation 11)}$$

The resultant is a change in the electric conductivity per unit concentration, that is, a concentration response constant. According to comparison of Equation 6, the concentration response constant K1/K2 in Example 1 is 1.16E-7 (SmL/mg), and the concentration response constant K1/K2 in Comparative Example 1 is 8.13E-11 (SmL/mg) to be three digits smaller than the former.

The measured value of the electric conductivity is far smaller than that in Example 1. Thus, a stable measurement is also difficult so that an accidental error is easily generated.

This would be because the membrane thickness of the phospholipid bilayer membrane is large so that sufficient ion channels are not formed up to the vicinity of the electrode by the labeled antigen.

It is therefore understood that the method according to Example 1 is superior.

(Example 2)

Next, a thin film having a hydrocarbon group $C_2F_5CH_2CH_2$ was formed as shown by Chemical Formula 1 by coating a 1 vol % of $C_6F_{13}CH_2CH_2SH$ solution in $CH_3CH_2OH$, and then drying the coated solution instead of the formation of the thin film 112 having the hydrocarbon group $C_4H_9$ as shown by Chemical Formula 1 by coating and drying the 1 vol % of $C_4H_9SH$ solution in $CH_3CH_2OH$ on the electrode film for the working electrode 351 in Example 1. In the same way as in Example 1, a liquid to be measured containing 800 ng/mL of an estradiol antigen was supplied thereto, and the electric conductivity was measured. The results are shown in Fig. 17. It is understood that when 1/σ and 1/C are used to convert Fig. 17, a linear relationship as shown in Fig. 18 is obtained. Consequently, it was verified that the labeled antigen undergoes Langmuir isothermal adsorption in accordance with the concentration thereof, and thus ion

channels are formed so that ions pass through the film, whereby a change in the electric conductivity, that is, an electric response is made.

According to comparison between the resultant linear relationship equation and Equation 6, the response constant K1 in Example 1 is 2.45E-05(S), and the response constant K1 in Example 2 is as large as 4.50E-05(S).

According to comparison between Equations 11 and 6, the concentration response constant K1/K2 in Example 1 is 1.16E-7 (SmL/mg), and the concentration response constant K1/K2 in Example 2 is as large as 1.98E-07 (SmL/mg).

This would be because the fluoroalkyl groups are exposed onto the thin film surface so that moieties not covered with the labeled antigen are larger in water repellency, and thus Na ions easily gather into the hydrophilic ion channels.

It is therefore understood that the method according to Example 2 is superior.

(Example 3)

Next, a thin film 112 having a hydrocarbon group $C_nH_{n2n+1}$ was formed as shown by Chemical Formula 1 by coating and drying a 1 vol % of $C_nH_{2n+1}SH$ (n = 2, 4, 8, 12, and 16) solution in $CH_3CH_2OH$ on the electrode film for the working electrode 351 as in Example 1. In the same way as in Example 1, a liquid to be measured containing 800 ng/mL of an estradiol antigen was supplied thereto, and the electric conductivity was measured. The results are shown in Fig. 19. It is understood that when 1/σ and 1/C are used to convert Fig. 19, a linear relationship as shown in Fig. 20 is obtained. Consequently, it was verified that the labeled antigen undergoes Langmuir isothermal adsorption in accordance with the concentration thereof, and thus ion channels are formed so that ions pass through the film, whereby a change in the electric conductivity, that is, an electric response is made.

As compared with the behavior in the case where n is 4 in Example 1, that in the case where n is 2 is substantially the same. At the high concentration range, the amount of adsorption becomes large, and thus it is presumed that ion channels in the thin film are broken so that the electric conductivity becomes large. When n is more than 12, the electric conductivity becomes small so that the chip does not substantially respond. This would be because the thickness of the film becomes large similarly to that of the phospholipid bilayer membrane in Comparative Example 1. Thus, the film thickness can be a thickness corresponding to C12 or less, for example, C4.

(Example 4)

Next, the working electrode in Example 1 was changed to an ITO film, and a monomolecular film 112 having a hydrocarbon group $C_4H_9$ was formed as shown by Chemical Formula 2 by coating and drying a 1 vol % of $C_4H_9SiCl_3$ solution in $C_8H_{18}$ on an electrode film for the working electrode 351. In the same way as in Example 1, a liquid to be measured containing 800 ng/mL of an estradiol antigen was supplied thereto, and the electric conductivity was measured. The results are shown in Fig. 19. It is understood that when 1/σ and 1/C are used to convert Fig. 17, a linear relationship as shown in Fig. 18 is obtained. Consequently, it was verified that the labeled antigen undergoes Langmuir isothermal adsorption in accordance with the concentration thereof, and thus ion channels are formed so that ions pass through the film, whereby a change in the electric conductivity, that is, an electric response is made.

According to comparison between the resultant linear relationship equation and Equation 6, the response constant K1 in Example 1 is 2.45E-05(S), and the response constant K1 in Example 4 is 1.14E-05(S) to be slightly smaller than the former.

According to comparison between Equations 11 and 6, the concentration response constant K1/K2 in Example 1 is 1.16E-7 (SmL/mg), and the concentration response constant K1/K2 in Example 4 is 7.35E-08 (SmL/mg) to be slightly smaller than the former.

On the other hand, these chips were immersed into the liquid to be measured for 6 hours, and then the same measurement was again made. In the case of Example 1, the results were varied while in the case of Example 4, the results were not varied.

The reason therefor would be as follows: the covalent, electrically-insulating siloxane groups are preset at the interface between the thin film and the substrate, and moieties not covered by the labeled antigen are larger in water repellency, so that Na ions easily gather into hydrophilic ion channels.

It is therefore understood that the method according to Example 4 is superior in endurance and measurement stability.

According to the measuring method and measuring device for measuring a biological substance provided by the present invention, a biological substance labeled with an ion conductive compound is adsorbed onto a thin film, which is formed on an electrode and has a hydrocarbon group, so that an ion channel is formed from the ion conductive group up to the upper of the electrode while penetrating through the thin film having the hydrocarbon group. Moreover, deterioration with time of the thin film having the hydrocarbon group is restrained. It is therefore possible to provide a measuring method, a measuring chip and a measuring device for a biological substance that are remarkably improved in electric responsibility and reliability.

DESCRIPTION OF REFERENCE SIGNS

1: Na ion
101: Na ion
201: Na ion
11: Thin film having hydrocarbon group
111: Thin film having hydrocarbon group
211: Thin film having hydrocarbon group
12: Hydrocarbon group
112: Hydrocarbon group
211: Hydrocarbon group
13: Ion channel
113: Ion channel
213: Ion channel
21: Biological substance having ion conductive group
22: Ion conductive group
122: Ion conductive group
232: Ion conductive group
120: Antigen
120A: Estradiol antigen
220A: Estradiol antigen
121: Labeled antigen
121A: Labeled antigen
122A: Raw material of ion conductive group
232A: Raw material of ion conductive group
123: Antigen region
223: Antigen region
130: Immobilized antibody
131: Complex of labeled antigen and immobilized antibody
132: Substrate
222: Substrate
132A: Polystyrene beads
223A: Polystyrene beads
132B: Brominated polystyrene
223B: Aminated polystyrene
133: Antibody region
233: Antibody region
130H: H chain
230H: H chain
130L: L chain
230L: L chain
130S: Disulfide bond
230S: Disulfide bond
135: Antibody (monovalent)
220: Immobilized antigen
221: Complex of labeled antibody and immobilized antigen
221A: Immobilized antigen
230: Antibody
231: Labeled antibody
231A: Labeled antibody
350: Measuring chip
450: Measuring chip
351: Substrate
451: Substrate
352: Working electrode
452: Working electrode

353: Pipe wall
453: Pipe wall
354: Liquid supplying section
454: Liquid supplying section
355: Capillary liquid absorbing section
455: Capillary liquid absorbing section
356: Electric wire section
456: Electric wire section
357: Complex of labeled antigen and immobilized antibody filling section
457: Complex of labeled antibody and immobilized antigen filling section
458: Counter electrode
360: Operating device
460: Operating device
361: Electric conductive moiety detecting section
461: Electric conductive moiety detecting section
362: Temperature detecting section
462: Temperature detecting section
363: Antigen concentration calculating section
463: Antibody concentration detecting section

## Claims

**1.** A chip for measuring the amount of a biological substance present in a liquid to be measured, comprising

(a) an immobilized antibody;
(b) a substance labeled with an ion conductive compound wherein the substance binds to the immobilized antibody; and
(c) metal electrodes comprising a working electrode and a counter electrode,

wherein the working electrode has, on its surface, a thin film comprising a linear fluoroalkyl group and the number of carbon atoms of the linear fluoroalkyl group is from 3 to 12.

**2.** The chip according to claim 1, wherein the thin film is formed by binding $CF_3(CF_2)_{n-2}(CH_2)_2$-$SiX_3$ wherein n is an integer of 2 to 11 and X is a halogen atom to the electrode.

**3.** The chip according to claim 1 or 2, wherein the substance or antibody is released from the immobilized antibody or immobilized substance due to the presence of the biological substance in the liquid to be measured, wherein the substance or antibody is labeled with the ion conductive compound;
an ion conductive compound moiety of the released substance or antibody labeled with the ion conductive compound is adsorbed onto the thin film formed on the surface of the working electrode; and
the amount of a change in the electric conductivity of the thin film that is induced by the adsorption of the ion conductive compound moiety is measured to determine the amount of the biological substance in the liquid to be measured.

**4.** The chip according to any one of claims 1-3, wherein the change in the amount of the electric conductivity is measured through the application of an alternating voltage or the application of an alternating voltage to which a direct voltage bias is added.

**5.** The chip according to any one of claims 1-4, wherein the immobilized antibody or the antibody labeled with the ion conductive compound is a monovalent antibody.

**6.** The chip according to any one of claims 1-5, wherein the immobilized antibody or the antibody labeled with the ion conductive compound is a monoclonal antibody.

**7.** The chip according to any one of claims 1-6, wherein the thin film is a monomolecular film.

**8.** The chip according to any one of claims 1-7, wherein the ion conductive compound is a compound having a crown

ether structure.

**9.** A device for measuring the amount of a biological substance in a liquid to be measured, comprising the chip according to any one of claims 1-8.

**10.** The device according to any one of claims 1-9, further comprising
an electric conductivity detecting section for detecting the electric conductivity of the thin film that is changed when an ion conductive compound moiety of the substance labeled with the ion conductive compound is adsorbed onto the working electrode; and
a substance concentration calculating section for calculating the amount of the substance in the liquid to be measured based on a change in the electric conductivity.

**11.** A method for measuring the amount of a biological substance in a liquid to be measured, comprising

(a) allowing an immobilized antibody to which a substance labeled with an ion conductive compound binds to contact with the biological substance in the liquid to be measured;
(b) allowing an ion conductive compound moiety of the substance labeled with the ion conductive compound which is released by a replacement reaction between the biological substance and the substance labeled with the ion conductive compound to be adsorbed onto a thin film formed on a surface of a metal electrode and containing a linear fluoroalkyl group, wherein the number of carbon atoms of the linear fluoroalkyl group is from 3 to 12; and
(c) measuring a change in the electric conductivity of the thin film.

**12.** The method according to claim 11, wherein the thin film is formed by binding $CF_3(CF_2)_{n-2}(CH_2)_2\text{-}SiX_3$ wherein n is an integer of 2 to 11 and X is a halogen atom to the electrode.

**Patentansprüche**

**1.** Chip zum Messen der Menge einer biologischen Substanz, die in einer zu messenden Flüssigkeit vorliegt, umfassend

(a) einen immobilisierten Antikörper,
(b) eine Substanz, gelabelt mit einer ionenleitenden Verbindung, wobei die Substanz an den immobilisierten Antikörper bindet, und
(c) Metallelektroden, umfassend eine Arbeitselektrode und eine Gegenelektrode,

wobei die Arbeitselektrode auf deren Oberfläche einen dünnen Film aufweist, umfassend eine lineare Fluoralkylgruppe und wobei die Anzahl an Kohlenstoffatomen der linearen Fluoralkylgruppe von 3 bis 12 ist.

**2.** Chip gemäß Anspruch 1, wobei der dünne Film durch Binden von $CF_3(CF_2)_{n-2}(CH_2)_2\text{-}SiX_3$, wobei n eine ganze Zahl von 2 bis 11 ist und X ein Halogenatom ist, an die Elektrode gebildet wird.

**3.** Chip gemäß Anspruch 1 oder 2, wobei die Substanz oder der Antikörper von dem immobilisierten Antikörper oder der immobilisierten Substanz aufgrund der Gegenwart der biologischen Substanz in der zu messenden Flüssigkeit freigelassen wird, wobei die Substanz oder der Antikörper mit der ionenleitfähigen Verbindung gelabelt ist,
wobei eine ionenleitfähige Verbindungseinheit der freigelassenen Substanz oder des Antikörpers, gelabelt mit der ionenleitfähigen Verbindung, auf dem Film, gebildet auf der Oberfläche der Arbeitselektrode, adsorbiert wird, und die Menge einer Änderung in der elektrischen Leitfähigkeit des dünnen Films, welche durch die Adsorption der ionenleitfähigen Verbindungseinheit induziert wird, gemessen wird, um die Menge der biologischen Substanz in der zu messenden Flüssigkeit zu bestimmen.

**4.** Chip gemäß einem der Ansprüche 1 - 4, wobei die Änderung in der Menge der elektrischen Leitfähigkeit durch das Anlegen einer Wechselspannung oder das Anlegen einer Wechselspannung, an welche eine direkte Vorspannung gegeben wird, gemessen wird.

**5.** Chip gemäß einem der Ansprüche 1 - 4, wobei der immobilisierte Antikörper oder der Antikörper, gelabelt mit der ionenleitfähigen Verbindung, ein monovalenter Antikörper ist.

**6.** Chip gemäß einem der Ansprüche 1 - 5, wobei der immobilisierte Antikörper oder der Antikörper, gelabelt mit der ionenleitfähigen Verbindung, ein monoklonaler Antikörper ist.

**7.** Chip gemäß einem der Ansprüche 1 - 6, wobei der dünne Film ein monomolekularer Film ist.

**8.** Chip gemäß einem der Ansprüche 1 - 7, wobei die ionenleitfähige Verbindung eine Verbindung mit einer Kronenetherstruktur ist.

**9.** Vorrichtung zum Messen der Menge einer biologischen Substanz in einer zu messenden Flüssigkeit, umfassend den Chip gemäß einem der Ansprüche 1-8.

**10.** Vorrichtung gemäß einem der Ansprüche 1 - 9, weiter umfassend einen elektrische Leitfähigkeit detektierenden Abschnitt zum Detektieren der elektrischen Leitfähigkeit des dünnen Films, welche sich ändert, wenn eine ionenleitfähige Verbindungseinheit der Substanz, gelabelt mit der ionenleitfähigen Verbindung, auf der Arbeitselektrode adsorbiert wird, und
einen Substanzkonzentration-berechnenden Abschnitt zum Berechnen der Menge der Substanz in der zu messenden Flüssigkeit, basierend auf einer Änderung in der elektrischen Leitfähigkeit.

**11.** Verfahren zum Messen der Menge einer biologischen Substanz in einer zu messenden Flüssigkeit, umfassend

(a) das Zulassen, daß ein immobilisierter Antikörper, an welchen eine Substanz, gelabelt mit einer ionenleitfähigen Verbindung, bindet, die biologische Substanz in der zu messenden Flüssigkeit kontaktiert,
(b) das Zulassen, daß eine ionenleitfähige Verbindungseinheit der Substanz, gelabelt mit der ionenleitfähigen Verbindung, welche durch eine Verdrängungsreaktion zwischen der biologischen Substanz und der Substanz, gelabelt mit der ionenleitfähigen Verbindung, auf einem dünnen Film, gebildet auf einer Oberfläche einer Metallelektrode und enthaltend eine lineare Fluoralkylgruppe, wobei die Anzahl an Kohlenstoffatomen der linearen Fluoralkylgruppe von 3 bis 12 ist, adsorbiert wird, und
(c) das Messen einer Änderung in der elektrischen Leitfähigkeit des dünnen Films.

**12.** Verfahren gemäß Anspruch 11, wobei der dünne Film durch Binden von $CF_3(CF_2)_{n-2}(CH_2)_2\text{-}SiX_3$, wobei n eine ganze Zahl von 2 bis 11 ist und X ein Halogenatom ist, an die Elektrode gebildet wird.

**Revendications**

**1.** Puce pour mesurer la quantité d'une substance biologique présente dans un liquide à mesurer, comprenant :

(a) un anticorps immobilisé ;
(b) une substance marquée avec un composé conducteur d'ions, dans laquelle la substance se lie à l'anticorps immobilisé ; et
(c) des électrodes métalliques comprenant une électrode de travail et une contre-électrode,

dans laquelle l'électrode de travail présente, sur sa surface, une couche mince comprenant un groupe fluoroalkyle linéaire, et le nombre d'atomes de carbone du groupe fluoroalkyle linéaire est de 3 à 12.

**2.** Puce selon la revendication 1, dans laquelle la couche mince est formée par la liaison de $CF_3(CF_2)_{n.2}(CH_2)_2\text{-}SiX_3$, dans laquelle n est un nombre entier allant de 2 à 11 et X est un atome d'halogène à l'électrode.

**3.** Puce selon la revendication 1 ou 2, dans laquelle la substance ou l'anticorps est libéré à partir de l'anticorps immobilisé ou de la substance immobilisée en raison de la présence de la substance biologique dans le liquide à mesurer, dans laquelle la substance ou l'anticorps est marqué avec le composé conducteur d'ions :

un fragment de composé conducteur d'ions de la substance ou de l'anticorps libéré marqué avec le composé conducteur d'ions est adsorbé sur la couche mince formée sur la surface de l'électrode de travail ; et
la quantité de changement de conductivité électrique de la couche mince qui est induite par l'adsorption du fragment de composé conducteur d'ions est mesurée afin de déterminer la quantité de la substance biologique dans le liquide à mesurer.

4. Puce selon l'une quelconque des revendications 1 à 3, dans laquelle le changement de la quantité de la conductivité électrique est mesuré par l'application d'une tension alternative ou l'application d'une tension alternative à laquelle une tension continue de polarisation est ajoutée.

5. Puce selon l'une quelconque des revendications 1 à 4, dans laquelle l'anticorps immobilisé ou l'anticorps marqué avec le composé conducteur d'ions est un anticorps monovalent.

6. Puce selon l'une quelconque des revendications 1 à 5, dans laquelle l'anticorps immobilisé ou l'anticorps marqué avec le composé conducteur d'ions est un anticorps monoclonal.

7. Puce selon l'une quelconque des revendications 1 à 6, dans laquelle la couche mince est un film monomoléculaire.

8. Puce selon l'une quelconque des revendications 1 à 7, dans laquelle le composé conducteur d'ions est un composé ayant une structure d'éther couronne.

9. Dispositif de mesure de la quantité d'une substance biologique dans un liquide à mesurer, comprenant la puce selon l'une quelconque des revendications 1 à 8.

10. Dispositif selon l'une quelconque des revendications 1 à 9, comprenant en outre :

    une section de détection de la conductivité électrique servant à détecter la conductivité électrique de la couche mince, qui change quand un fragment de composé conducteur d'ions de la substance marquée avec le composé conducteur d'ions est adsorbé sur l'électrode de travail ; et
    une section calcul de la concentration en substance servant à calculer la quantité de la substance dans le liquide à mesurer sur la base d'un changement de la conductivité électrique.

11. Procédé de mesure de la quantité d'une substance biologique dans un liquide à mesurer, comprenant :

    (a) le fait de permettre à un anticorps immobilisé auquel une substance marquée avec un composé conducteur d'ions est liée d'entrer en contact avec la substance biologique dans le liquide à mesurer ;
    (b) le fait de permettre à un fragment de composé conducteur d'ions de la substance marquée avec le composé conducteur d'ions qui est libérée par une réaction de remplacement entre la substance biologique et la substance marquée avec le composé conducteur d'ions d'être adsorbé sur une couche mince formée sur une surface d'une électrode métallique et contenant un groupe fluoroalkyle linéaire, dans lequel le nombre d'atomes de carbone du groupe fluoroalkyle linéaire est de 3 à 12 ; et
    (c) la mesure d'un changement de la conductivité électrique de la couche mince.

12. Procédé selon la revendication 11, dans lequel la couche mince est formée par la liaison de $CF_3(CF_2)_{n.2}(CH_2)_2$-$SiX_3$, dans lequel n est un nombre entier allant de 2 à 11 et X est un atome d'halogène à l'électrode.

Fig.1

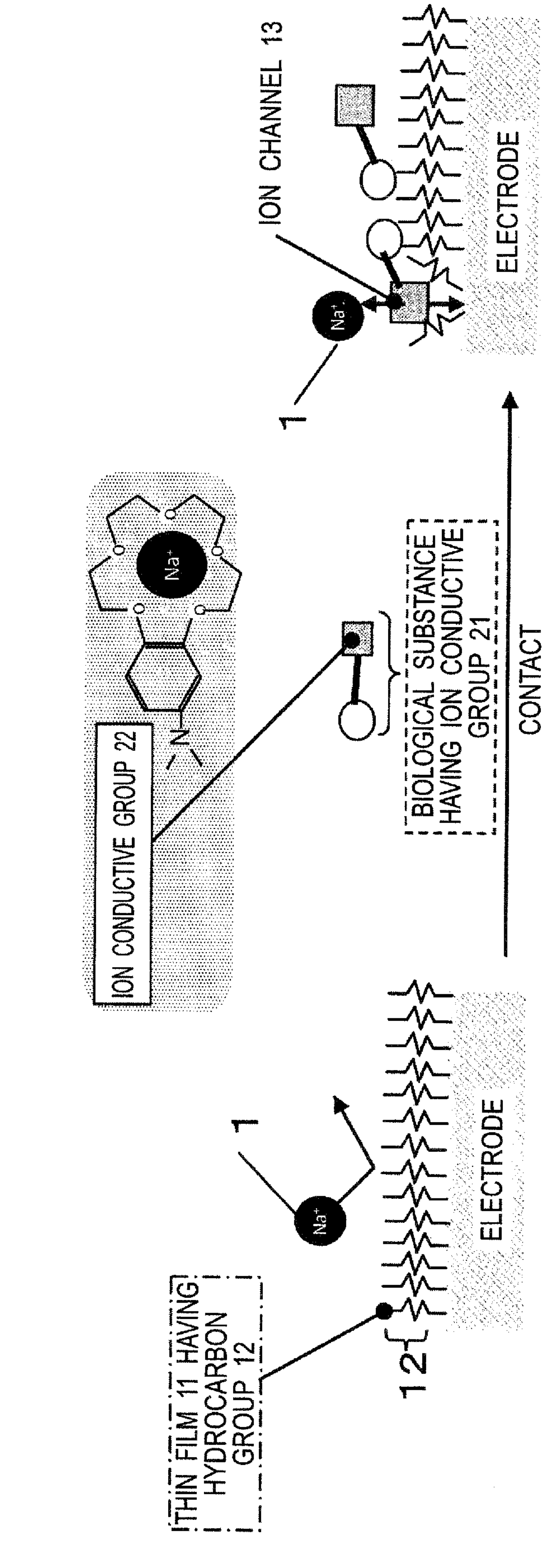
THIN FILM 11 HAVING HYDROCARBON GROUP 12
12
Na+
1
ELECTRODE
ION CONDUCTIVE GROUP 22
Na+
N
BIOLOGICAL SUBSTANCE HAVING ION CONDUCTIVE GROUP 21
CONTACT
1
Na+
ION CHANNEL 13
ELECTRODE

*Fig.2*

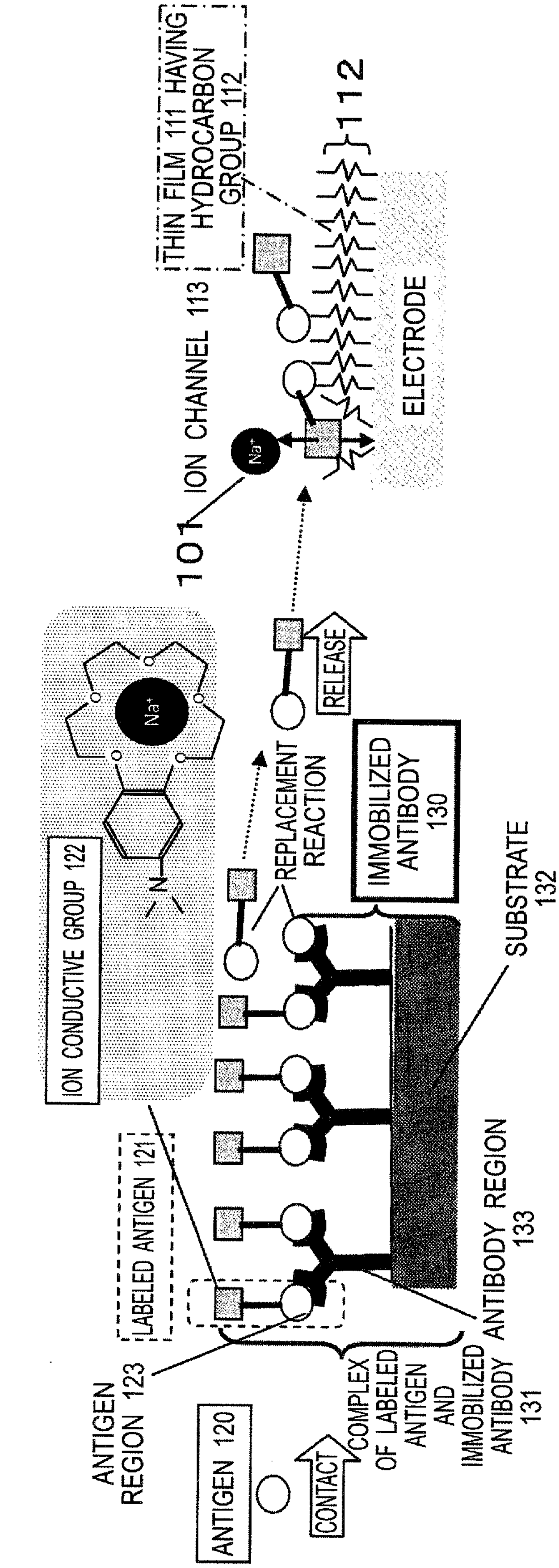
ANTIGEN 120
CONTACT
ANTIGEN REGION 123
COMPLEX OF LABELED ANTIGEN AND IMMOBILIZED ANTIBODY 131
LABELED ANTIGEN 121
ANTIBODY REGION 133
ION CONDUCTIVE GROUP 122
SUBSTRATE 132
REPLACEMENT REACTION
IMMOBILIZED ANTIBODY 130
RELEASE
Na+
101
ION CHANNEL 113
THIN FILM 111 HAVING HYDROCARBON GROUP 112
112
ELECTRODE

*Fig.3*

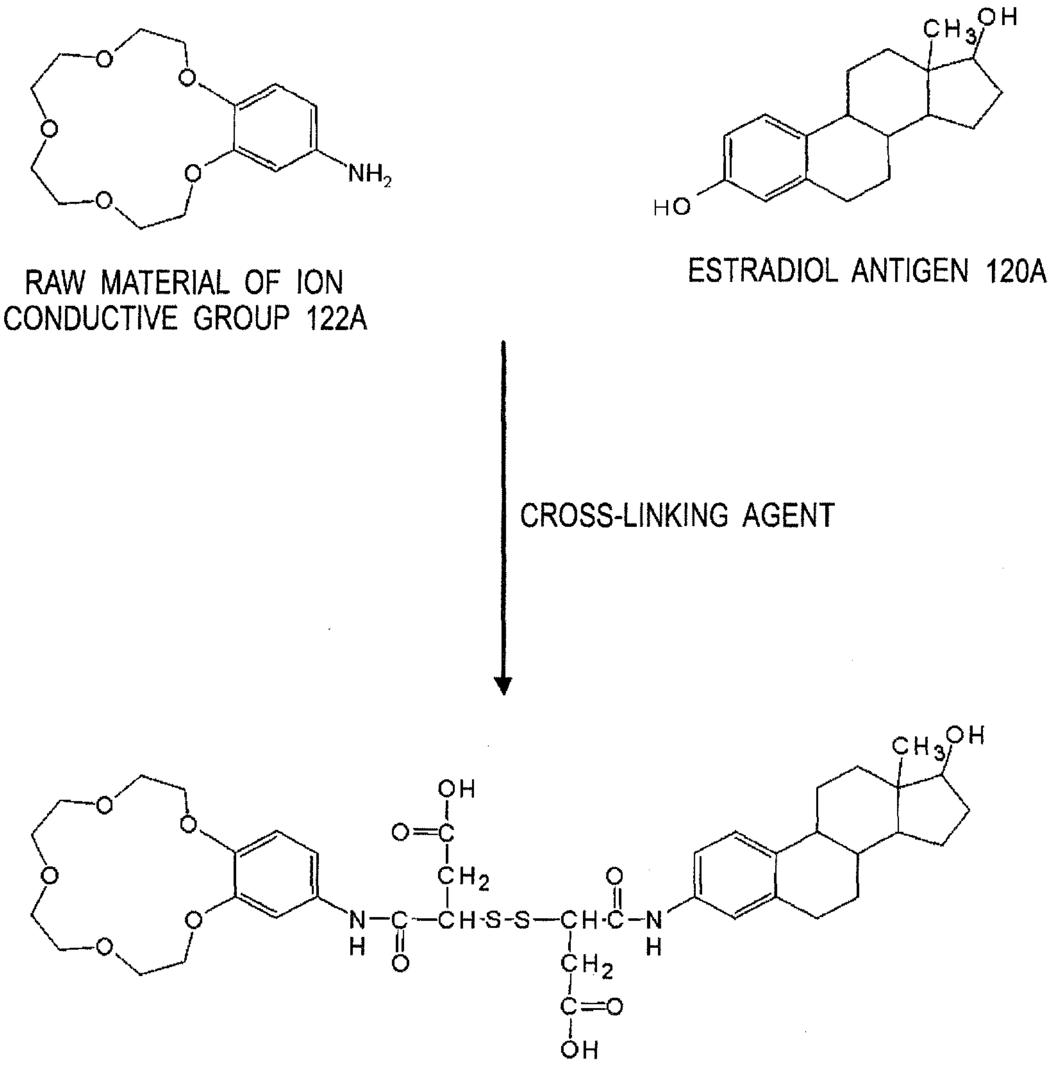


RAW MATERIAL OF ION CONDUCTIVE GROUP 122A

ESTRADIOL ANTIGEN 120A

LABELED ANTIGEN 121A

Fig.4

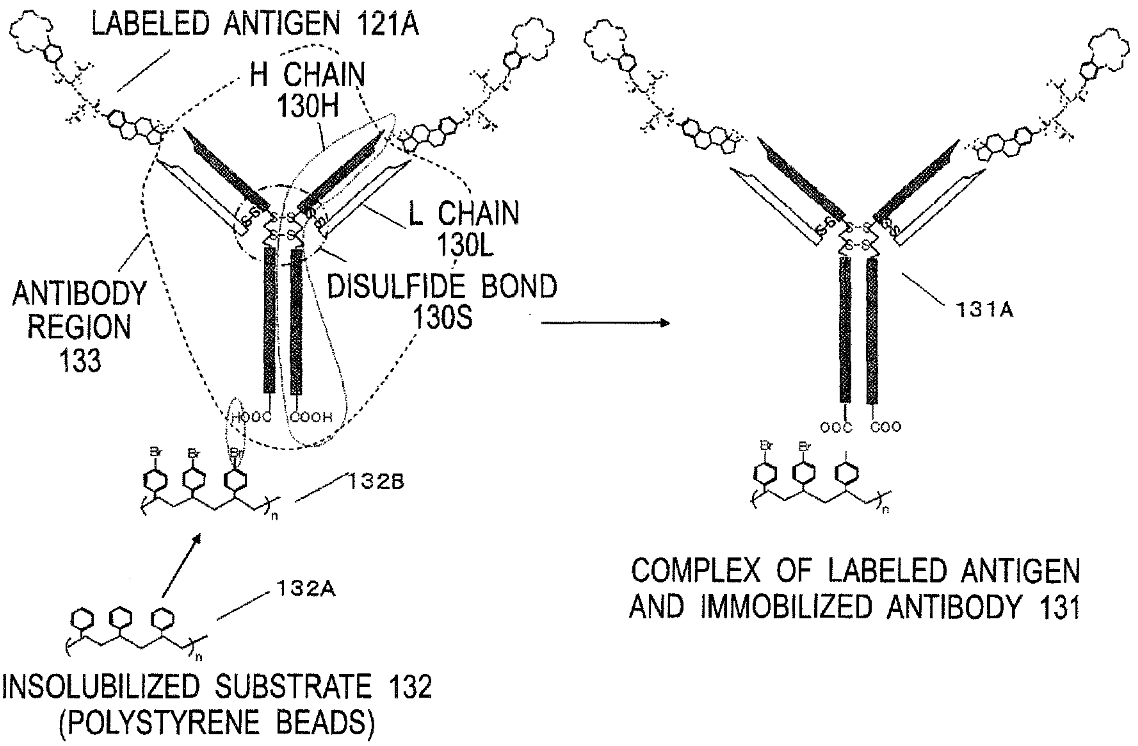
LABELED ANTIGEN 121A
H CHAIN
130H
L CHAIN
130L
ANTIBODY
REGION
133
DISULFIDE BOND
130S
HOOC
COOH
Br
132B
132A
131A
OOC
COO
COMPLEX OF LABELED ANTIGEN
AND IMMOBILIZED ANTIBODY 131
INSOLUBILIZED SUBSTRATE 132
(POLYSTYRENE BEADS)

*Fig.5*

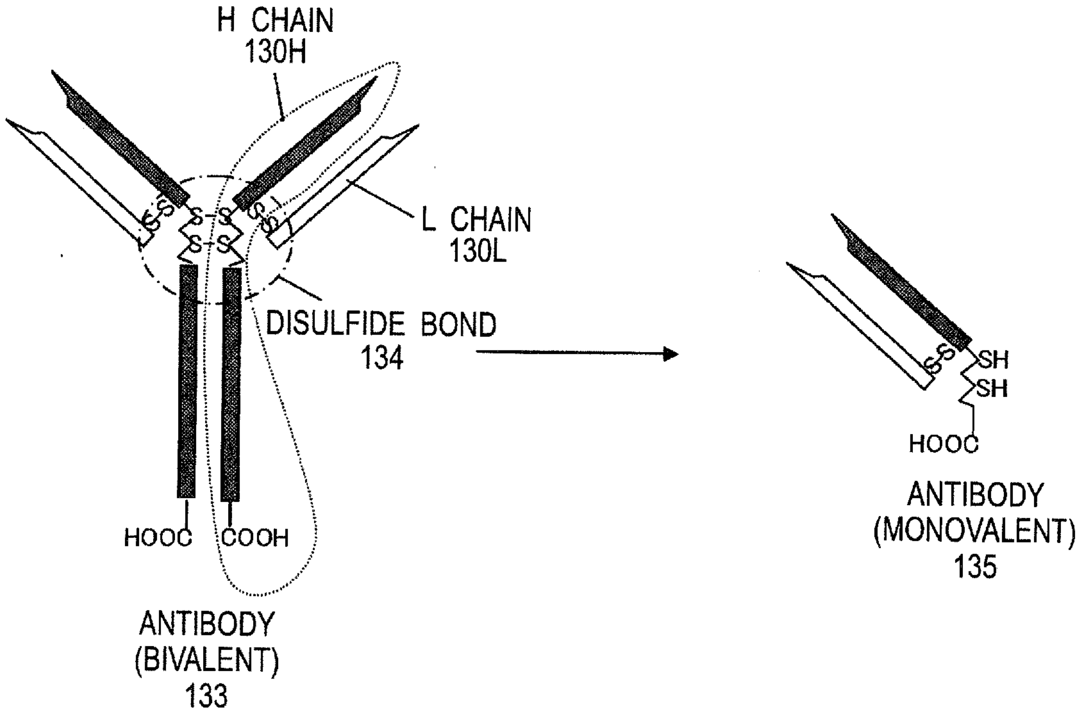
H CHAIN
130H
L CHAIN
130L
S-S
S-S
S-S
S-S
DISULFIDE BOND
134
HOOC
COOH
ANTIBODY
(BIVALENT)
133
S-S
SH
SH
HOOC
ANTIBODY
(MONOVALENT)
135

Fig.6

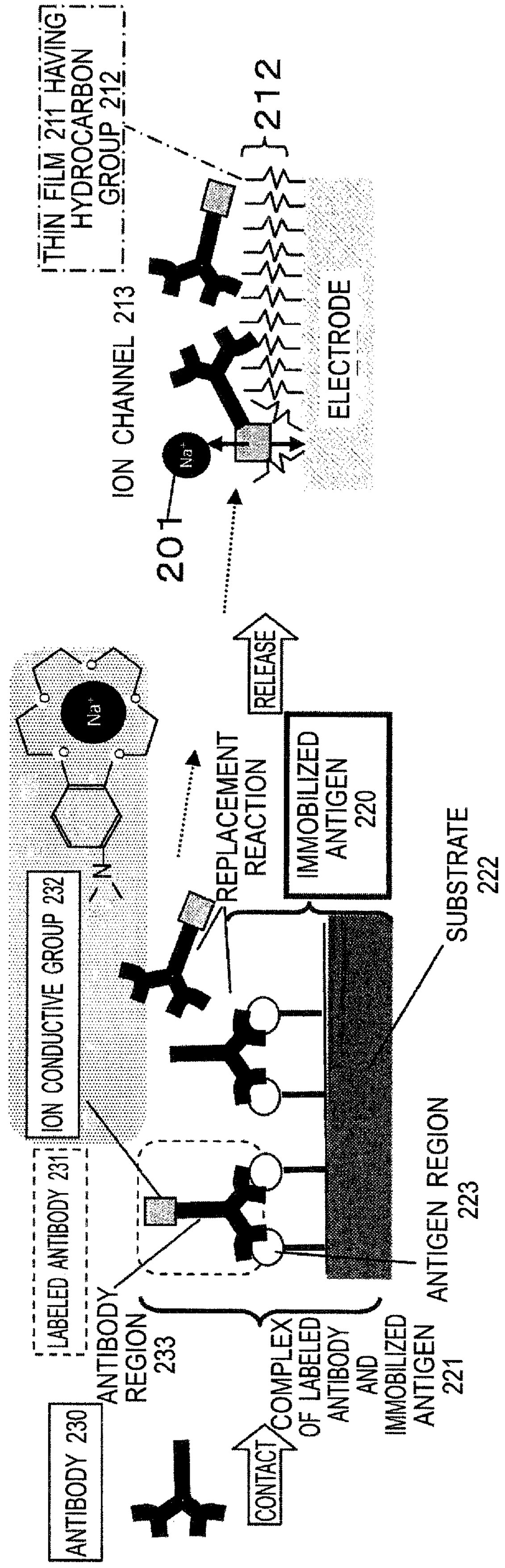
ANTIBODY 230
CONTACT
LABELED ANTIBODY 231
ANTIBODY REGION 233
COMPLEX OF LABELED ANTIBODY AND IMMOBILIZED ANTIGEN 221
ANTIGEN REGION 223
ION CONDUCTIVE GROUP 232
Na+
SUBSTRATE 222
REPLACEMENT REACTION
IMMOBILIZED ANTIGEN 220
RELEASE
201
Na+
ION CHANNEL 213
ELECTRODE
THIN FILM 211 HAVING HYDROCARBON GROUP 212
212

*Fig.7*

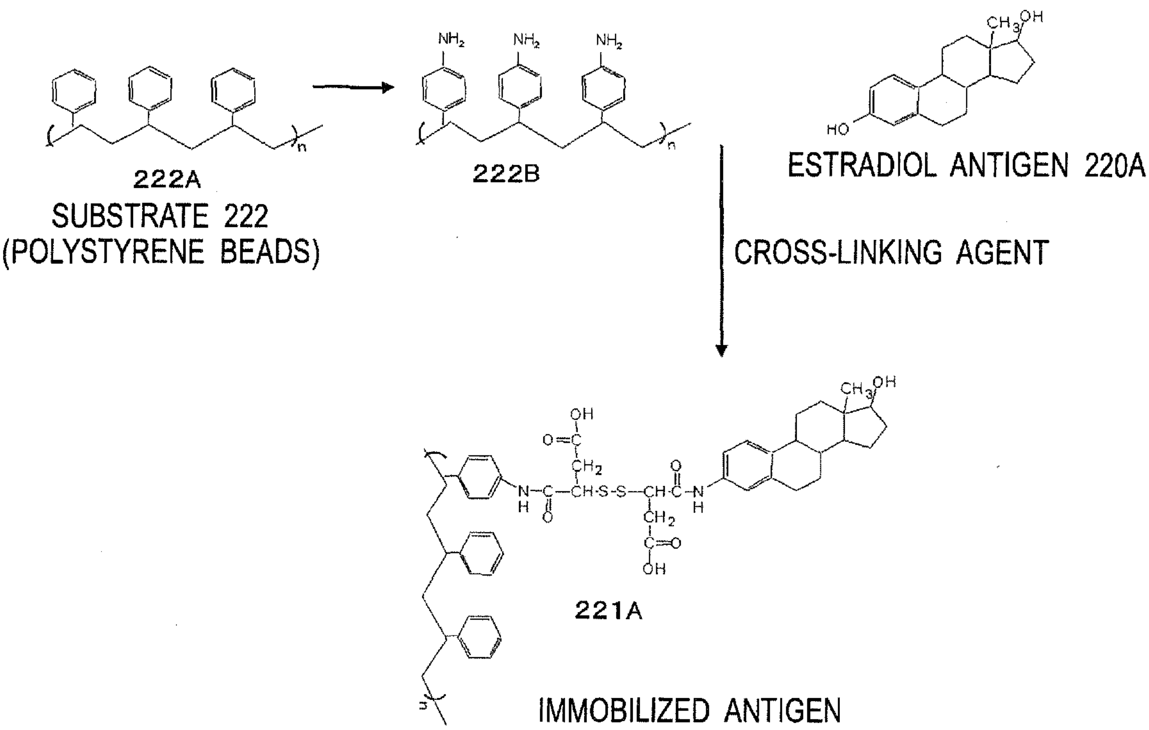


222A
SUBSTRATE 222
(POLYSTYRENE BEADS)

222B

ESTRADIOL ANTIGEN 220A

221A

IMMOBILIZED ANTIGEN

*Fig.8*

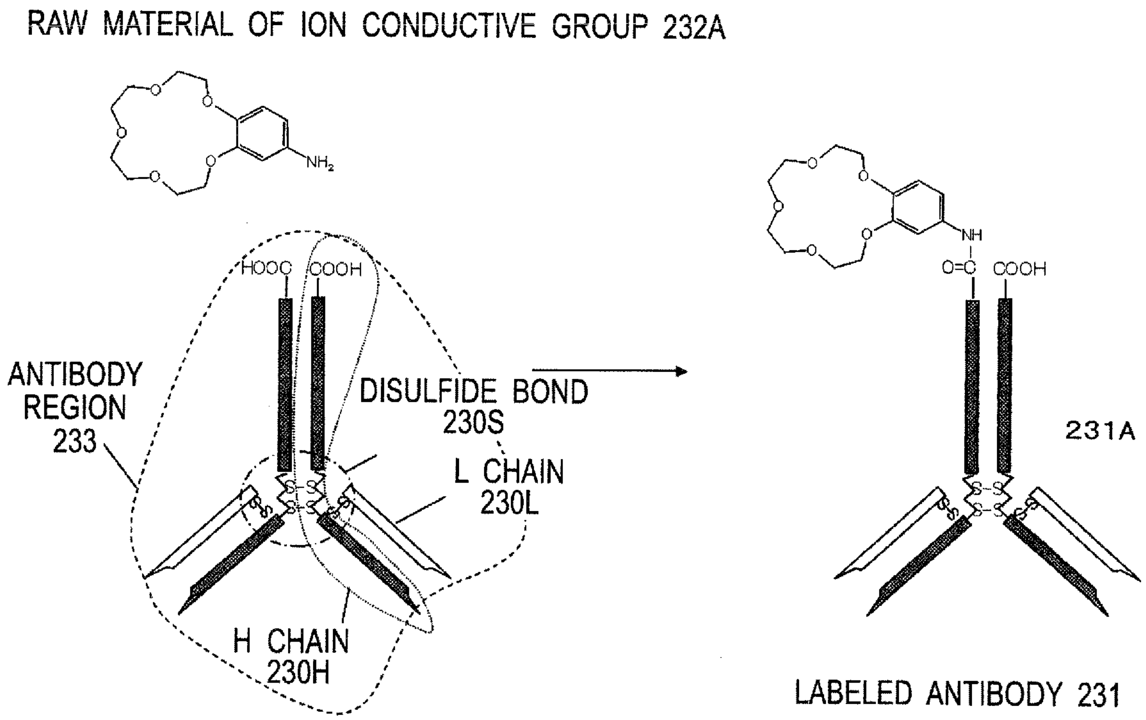
RAW MATERIAL OF ION CONDUCTIVE GROUP 232A
ANTIBODY REGION 233
DISULFIDE BOND 230S
L CHAIN 230L
H CHAIN 230H
231A
LABELED ANTIBODY 231

Fig.9

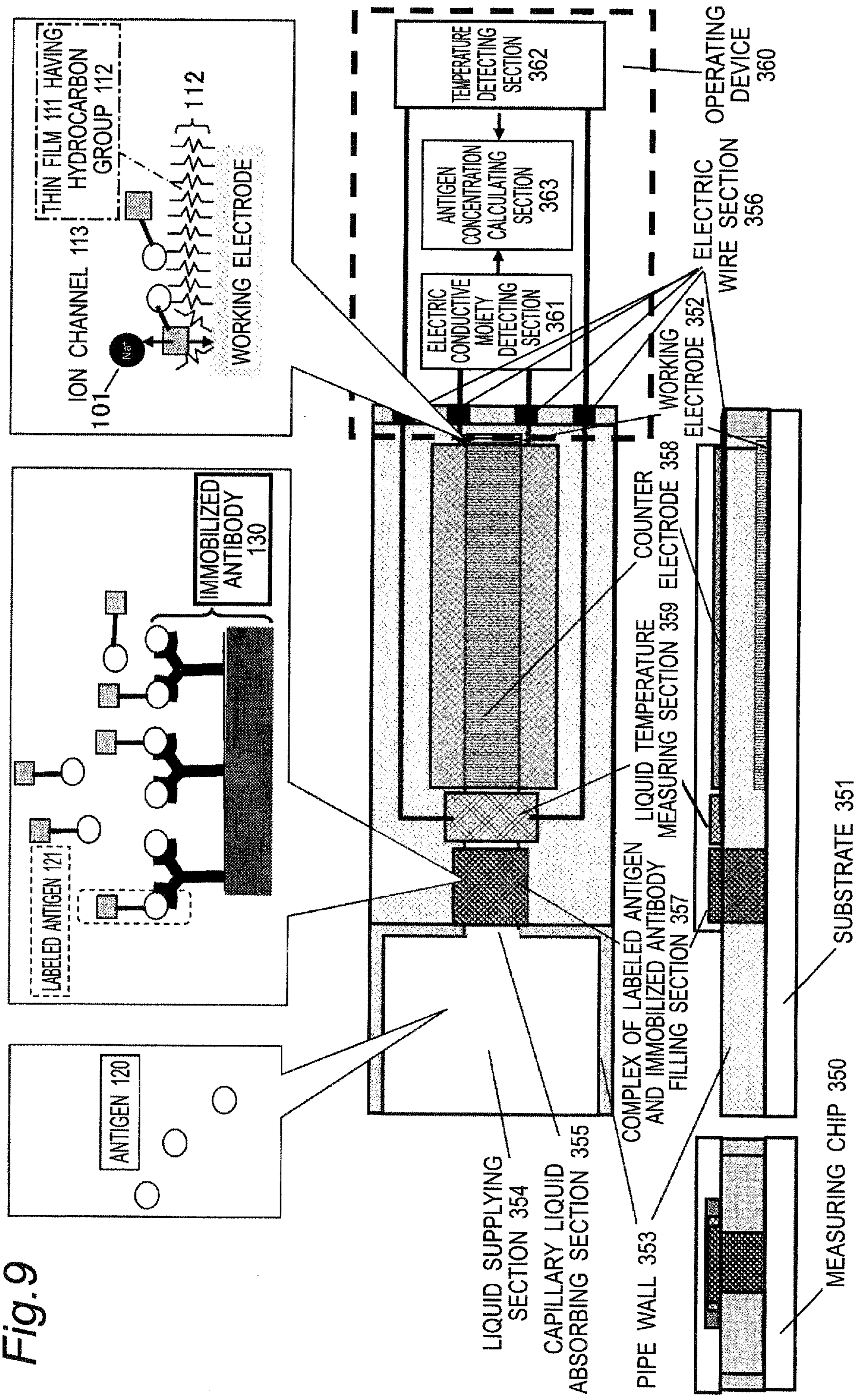
ANTIGEN 120
LABELED ANTIGEN 121
IMMOBILIZED ANTIBODY 130
ION CHANNEL 113
101
Na+
THIN FILM 111 HAVING HYDROCARBON GROUP 112
112
WORKING ELECTRODE
TEMPERATURE DETECTING SECTION 362
ANTIGEN CONCENTRATION CALCULATING SECTION 363
ELECTRIC CONDUCTIVE MOIETY DETECTING SECTION 361
OPERATING DEVICE 360
ELECTRIC WIRE SECTION 356
WORKING ELECTRODE 352
COUNTER ELECTRODE 358
LIQUID TEMPERATURE MEASURING SECTION 359
COMPLEX OF LABELED ANTIGEN AND IMMOBILIZED ANTIBODY FILLING SECTION 357
SUBSTRATE 351
MEASURING CHIP 350
PIPE WALL 353
CAPILLARY LIQUID ABSORBING SECTION 355
LIQUID SUPPLYING SECTION 354

Fig.10

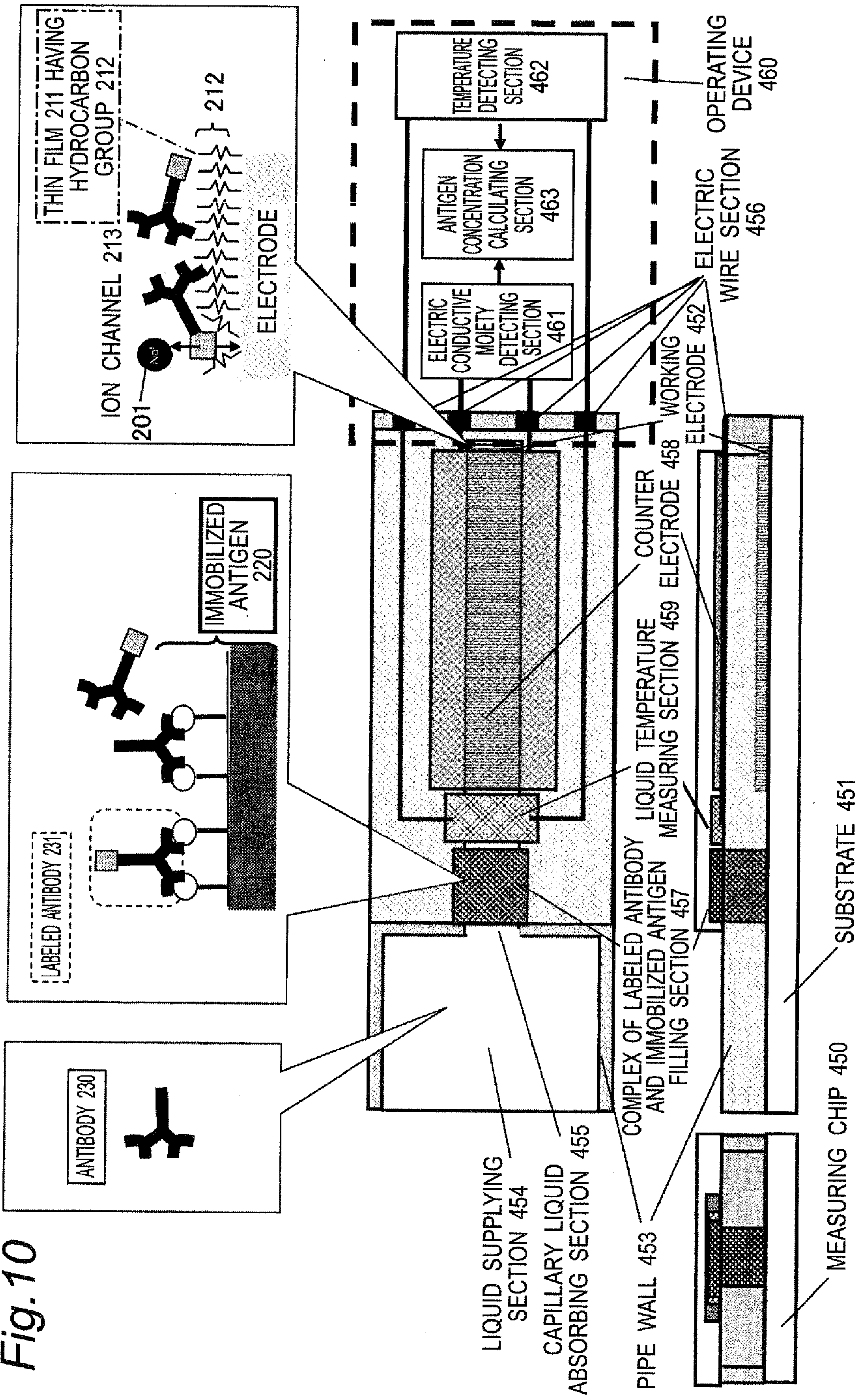

ANTIBODY 230
LABELED ANTIBODY 231
IMMOBILIZED ANTIGEN 220
201
ION CHANNEL 213
THIN FILM 211 HAVING HYDROCARBON GROUP 212
212
ELECTRODE
TEMPERATURE DETECTING SECTION 462
ANTIGEN CONCENTRATION CALCULATING SECTION 463
ELECTRIC CONDUCTIVE MOIETY DETECTING SECTION 461
OPERATING DEVICE 460
ELECTRIC WIRE SECTION 456
WORKING ELECTRODE 452
COUNTER ELECTRODE 458
LIQUID TEMPERATURE MEASURING SECTION 459
COMPLEX OF LABELED ANTIBODY AND IMMOBILIZED ANTIGEN FILLING SECTION 457
SUBSTRATE 451
MEASURING CHIP 450
LIQUID SUPPLYING SECTION 454
CAPILLARY LIQUID ABSORBING SECTION 455
PIPE WALL 453

*Fig.11*

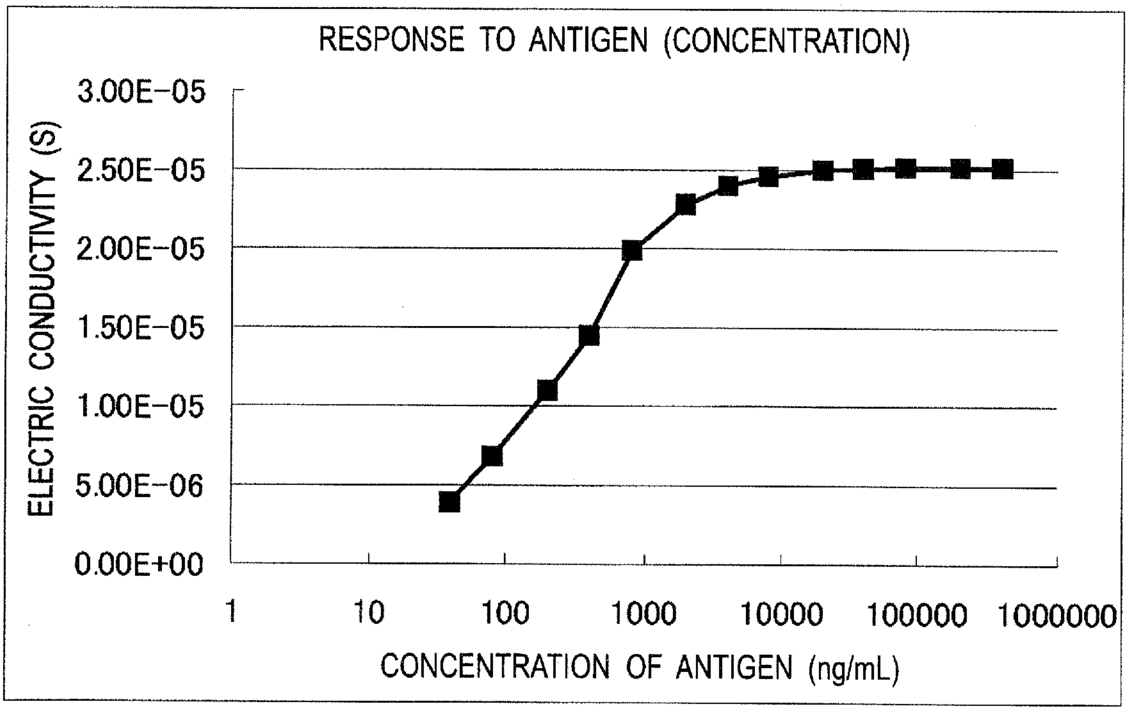
RESPONSE TO ANTIGEN (CONCENTRATION)
ELECTRIC CONDUCTIVITY (S)
3.00E-05
2.50E-05
2.00E-05
1.50E-05
1.00E-05
5.00E-06
0.00E+00
1
10
100
1000
10000
100000
1000000
CONCENTRATION OF ANTIGEN (ng/mL)

Fig.12

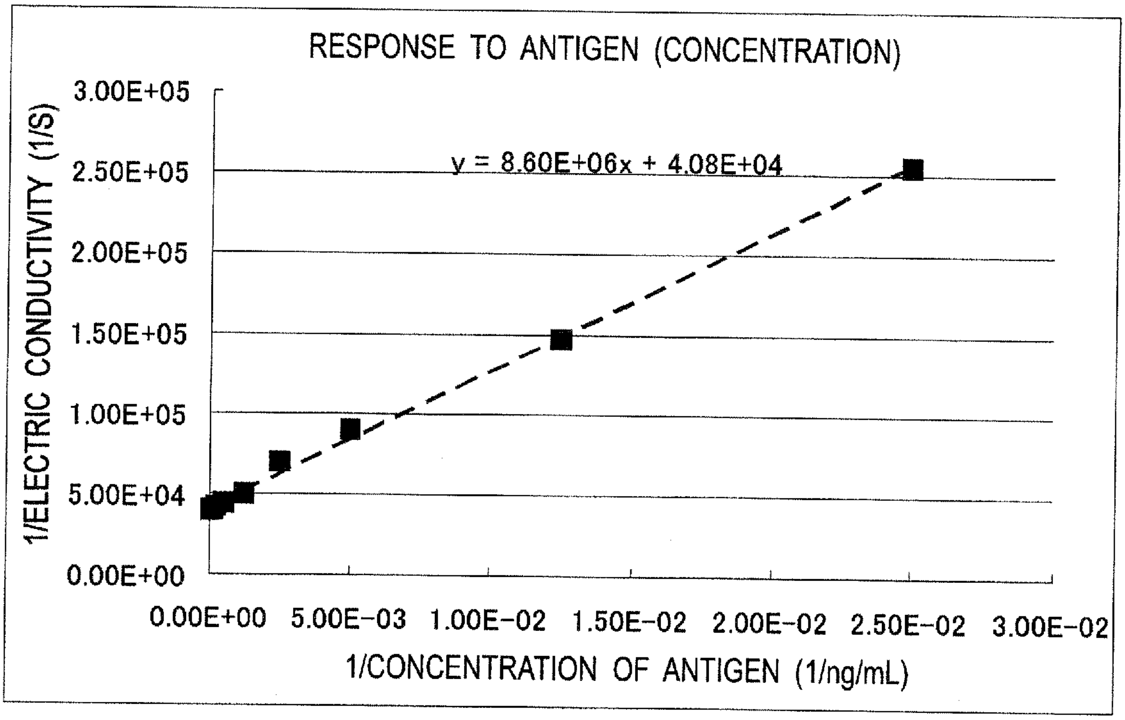
RESPONSE TO ANTIGEN (CONCENTRATION)
y = 8.60E+06x + 4.08E+04
1/ELECTRIC CONDUCTIVITY (1/S)
3.00E+05
2.50E+05
2.00E+05
1.50E+05
1.00E+05
5.00E+04
0.00E+00
0.00E+00
5.00E-03
1.00E-02
1.50E-02
2.00E-02
2.50E-02
3.00E-02
1/CONCENTRATION OF ANTIGEN (1/ng/mL)

Fig.13

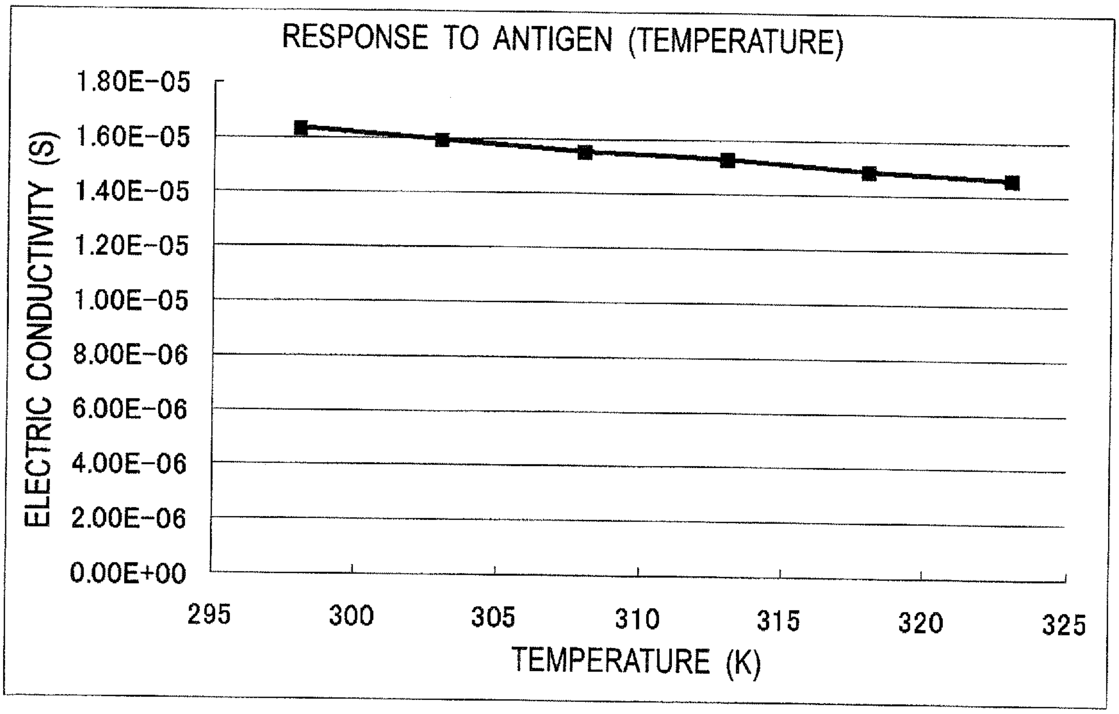
RESPONSE TO ANTIGEN (TEMPERATURE)
ELECTRIC CONDUCTIVITY (S)
1.80E-05
1.60E-05
1.40E-05
1.20E-05
1.00E-05
8.00E-06
6.00E-06
4.00E-06
2.00E-06
0.00E+00
295
300
305
310
315
320
325
TEMPERATURE (K)

*Fig.14*

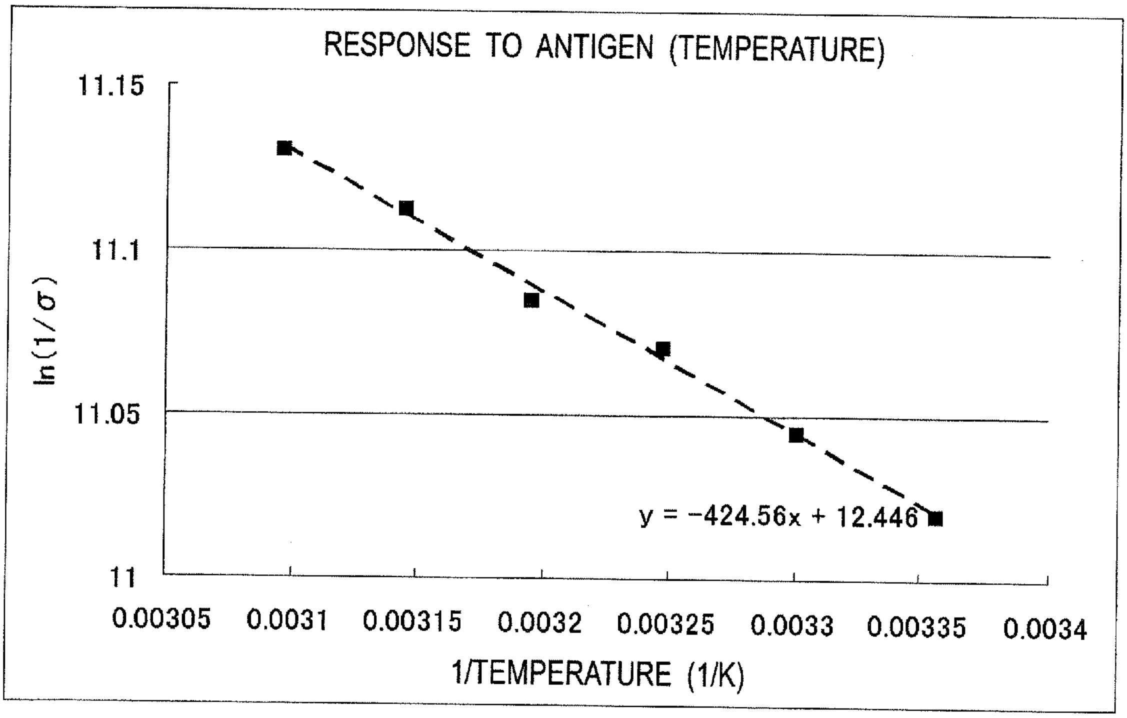
RESPONSE TO ANTIGEN (TEMPERATURE)
ln(1/σ)
11.15
11.1
11.05
11
y = −424.56x + 12.446
0.00305
0.0031
0.00315
0.0032
0.00325
0.0033
0.00335
0.0034
1/TEMPERATURE (1/K)

*Fig.15*

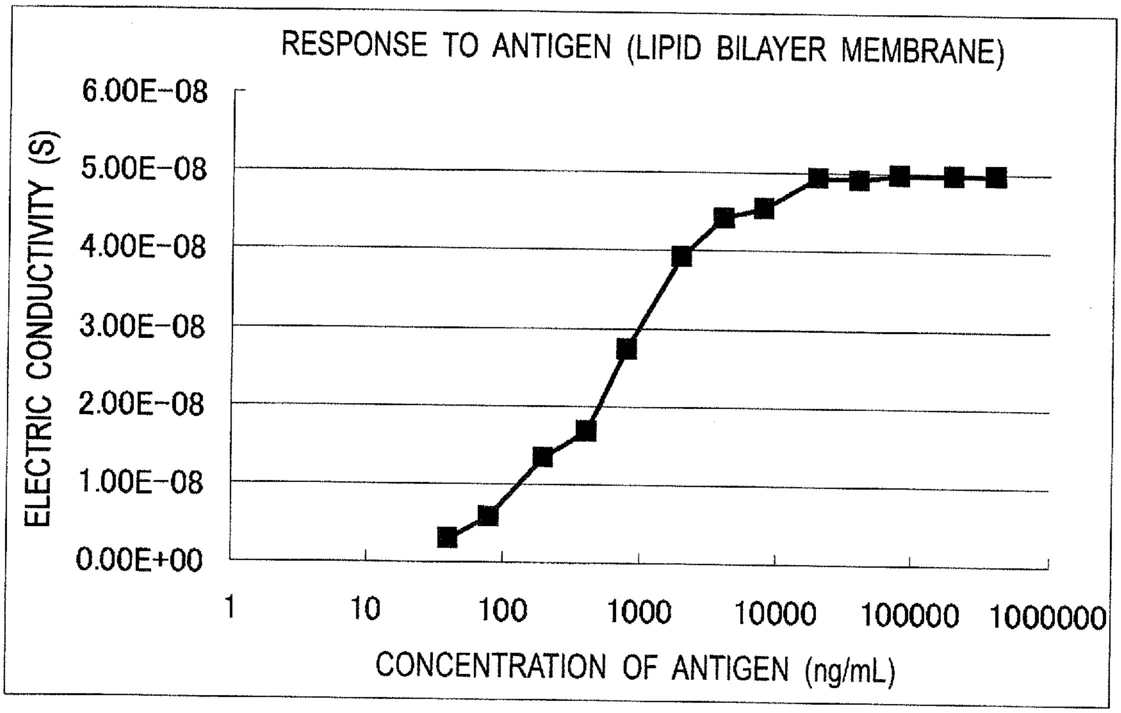
RESPONSE TO ANTIGEN (LIPID BILAYER MEMBRANE)
ELECTRIC CONDUCTIVITY (S)
6.00E−08
5.00E−08
4.00E−08
3.00E−08
2.00E−08
1.00E−08
0.00E+00
1
10
100
1000
10000
100000
1000000
CONCENTRATION OF ANTIGEN (ng/mL)

*Fig.16*

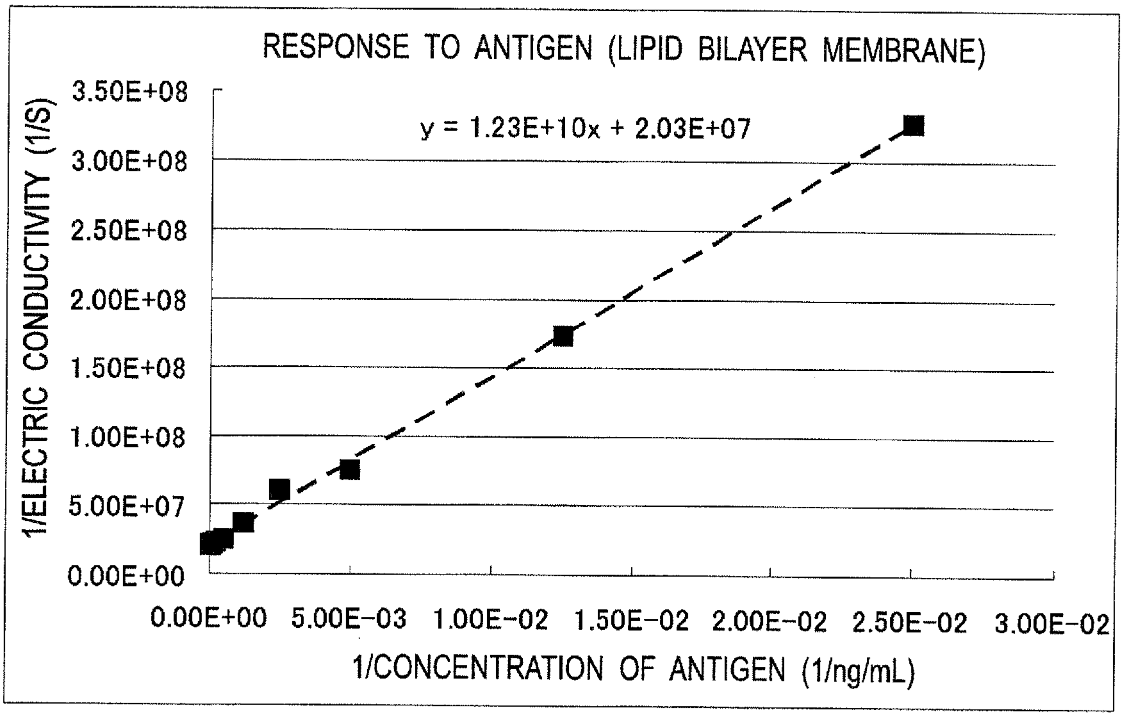
RESPONSE TO ANTIGEN (LIPID BILAYER MEMBRANE)
y = 1.23E+10x + 2.03E+07
1/ELECTRIC CONDUCTIVITY (1/S)
3.50E+08
3.00E+08
2.50E+08
2.00E+08
1.50E+08
1.00E+08
5.00E+07
0.00E+00
0.00E+00
5.00E-03
1.00E-02
1.50E-02
2.00E-02
2.50E-02
3.00E-02
1/CONCENTRATION OF ANTIGEN (1/ng/mL)

*Fig.17*

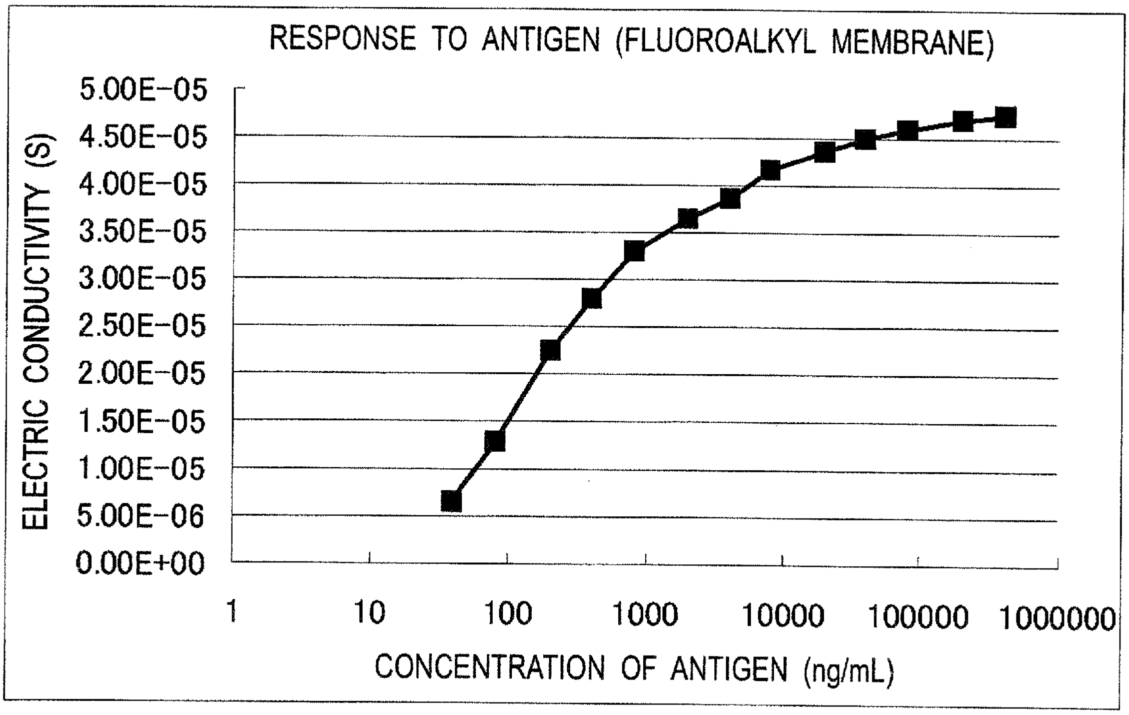
RESPONSE TO ANTIGEN (FLUOROALKYL MEMBRANE)
ELECTRIC CONDUCTIVITY (S)
5.00E-05
4.50E-05
4.00E-05
3.50E-05
3.00E-05
2.50E-05
2.00E-05
1.50E-05
1.00E-05
5.00E-06
0.00E+00
1
10
100
1000
10000
100000
1000000
CONCENTRATION OF ANTIGEN (ng/mL)

*Fig.18*

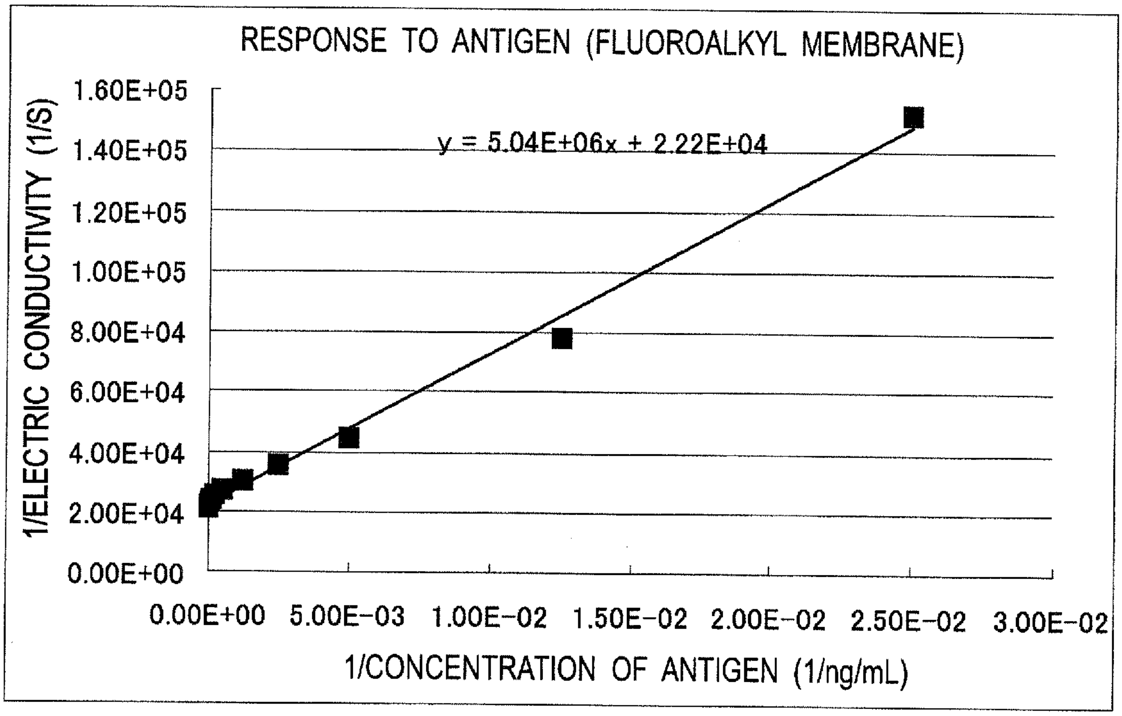
RESPONSE TO ANTIGEN (FLUOROALKYL MEMBRANE)
1/ELECTRIC CONDUCTIVITY (1/S)
1.60E+05
1.40E+05
1.20E+05
1.00E+05
8.00E+04
6.00E+04
4.00E+04
2.00E+04
0.00E+00
y = 5.04E+06x + 2.22E+04
0.00E+00
5.00E−03
1.00E−02
1.50E−02
2.00E−02
2.50E−02
3.00E−02
1/CONCENTRATION OF ANTIGEN (1/ng/mL)

*Fig.19*

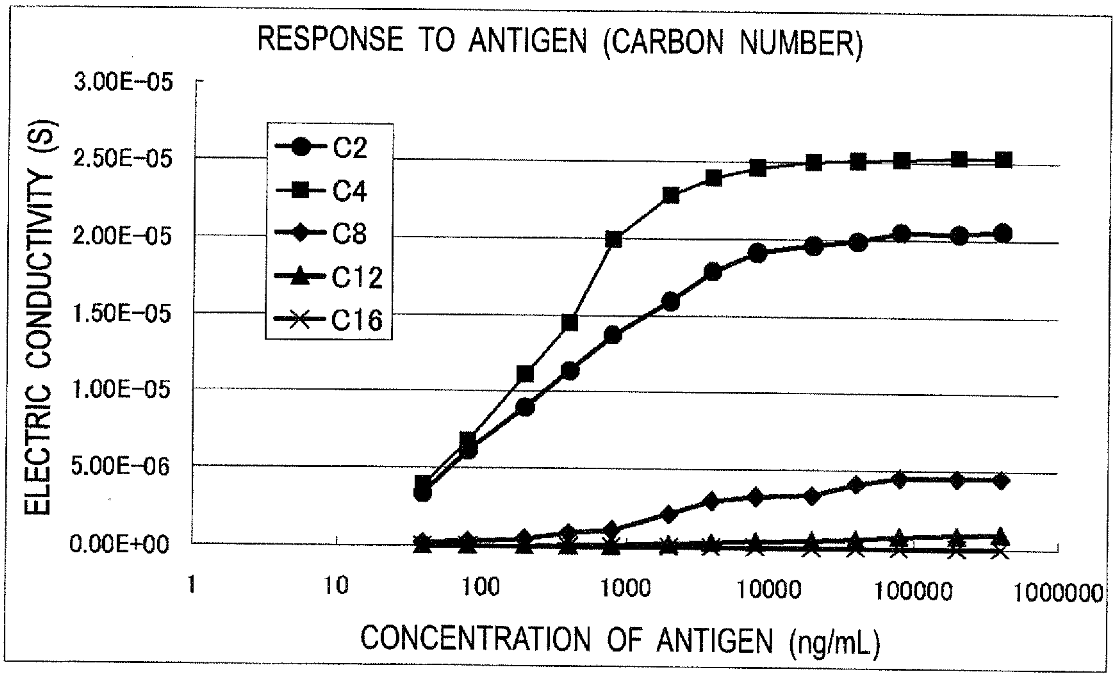
RESPONSE TO ANTIGEN (CARBON NUMBER)
ELECTRIC CONDUCTIVITY (S)
3.00E−05
2.50E−05
2.00E−05
1.50E−05
1.00E−05
5.00E−06
0.00E+00
C2
C4
C8
C12
C16
1
10
100
1000
10000
100000
1000000
CONCENTRATION OF ANTIGEN (ng/mL)

Fig.20

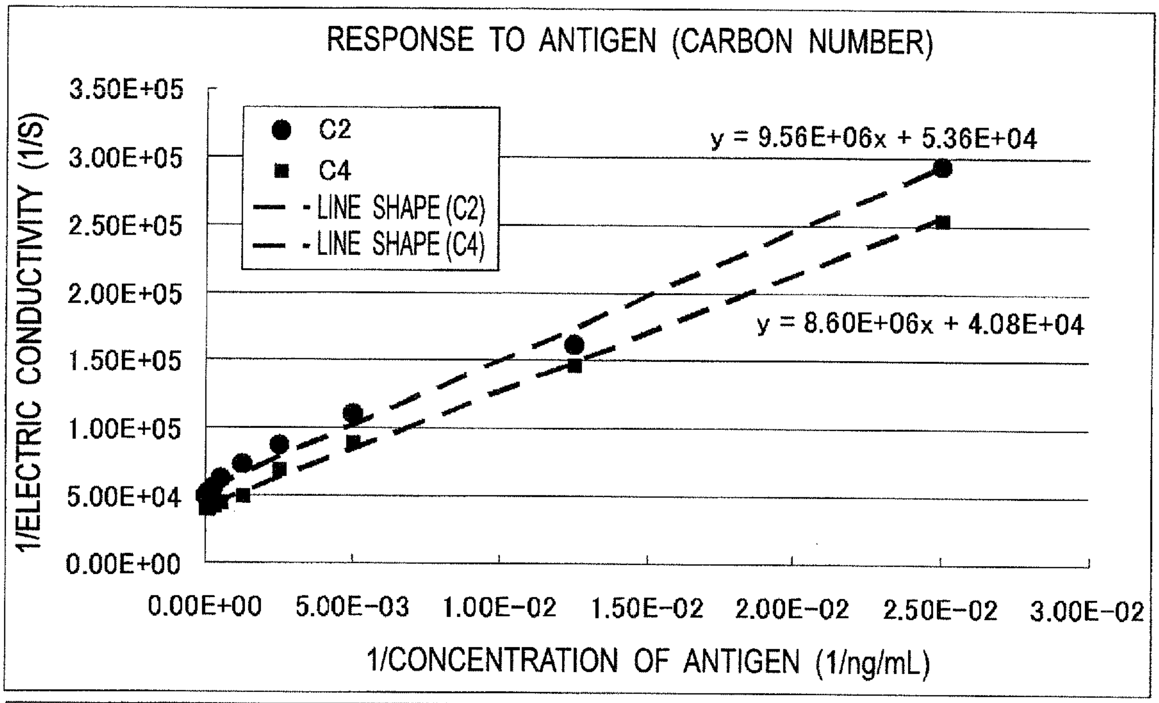
RESPONSE TO ANTIGEN (CARBON NUMBER)
1/ELECTRIC CONDUCTIVITY (1/S)
3.50E+05
3.00E+05
2.50E+05
2.00E+05
1.50E+05
1.00E+05
5.00E+04
0.00E+00
C2
C4
LINE SHAPE (C2)
LINE SHAPE (C4)
y = 9.56E+06x + 5.36E+04
y = 8.60E+06x + 4.08E+04
0.00E+00
5.00E-03
1.00E-02
1.50E-02
2.00E-02
2.50E-02
3.00E-02
1/CONCENTRATION OF ANTIGEN (1/ng/mL)

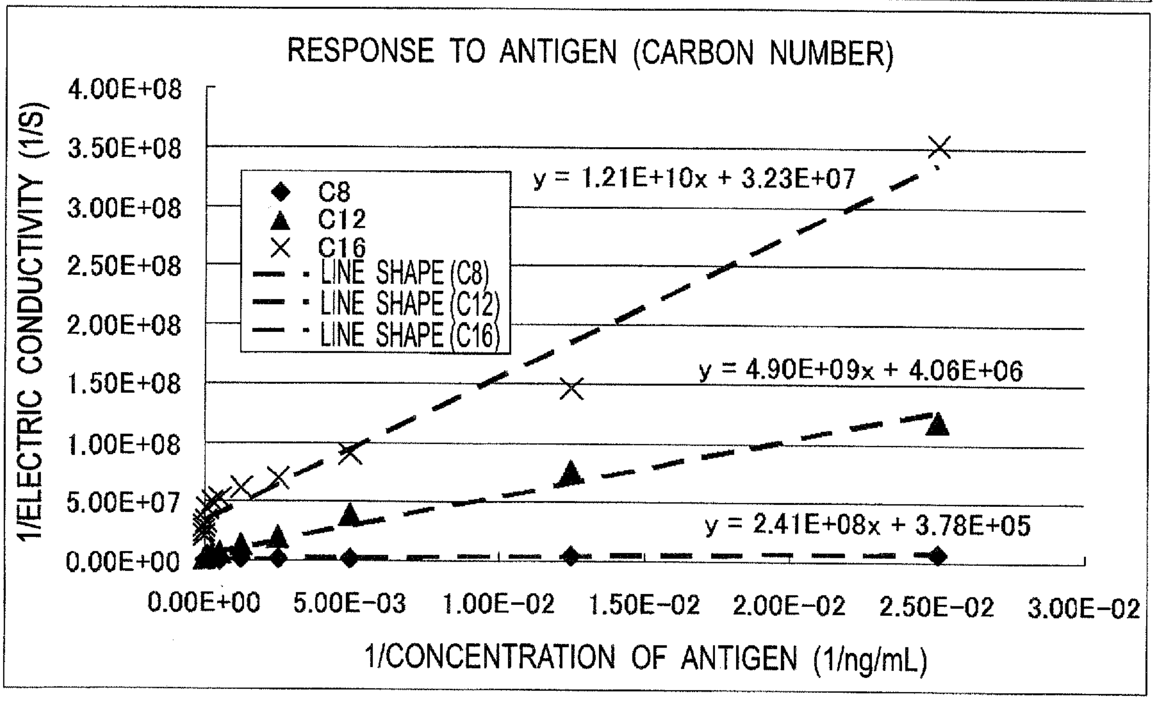
RESPONSE TO ANTIGEN (CARBON NUMBER)
1/ELECTRIC CONDUCTIVITY (1/S)
4.00E+08
3.50E+08
3.00E+08
2.50E+08
2.00E+08
1.50E+08
1.00E+08
5.00E+07
0.00E+00
C8
C12
C16
LINE SHAPE (C8)
LINE SHAPE (C12)
LINE SHAPE (C16)
y = 1.21E+10x + 3.23E+07
y = 4.90E+09x + 4.06E+06
y = 2.41E+08x + 3.78E+05
0.00E+00
5.00E-03
1.00E-02
1.50E-02
2.00E-02
2.50E-02
3.00E-02
1/CONCENTRATION OF ANTIGEN (1/ng/mL)

*Fig.21*

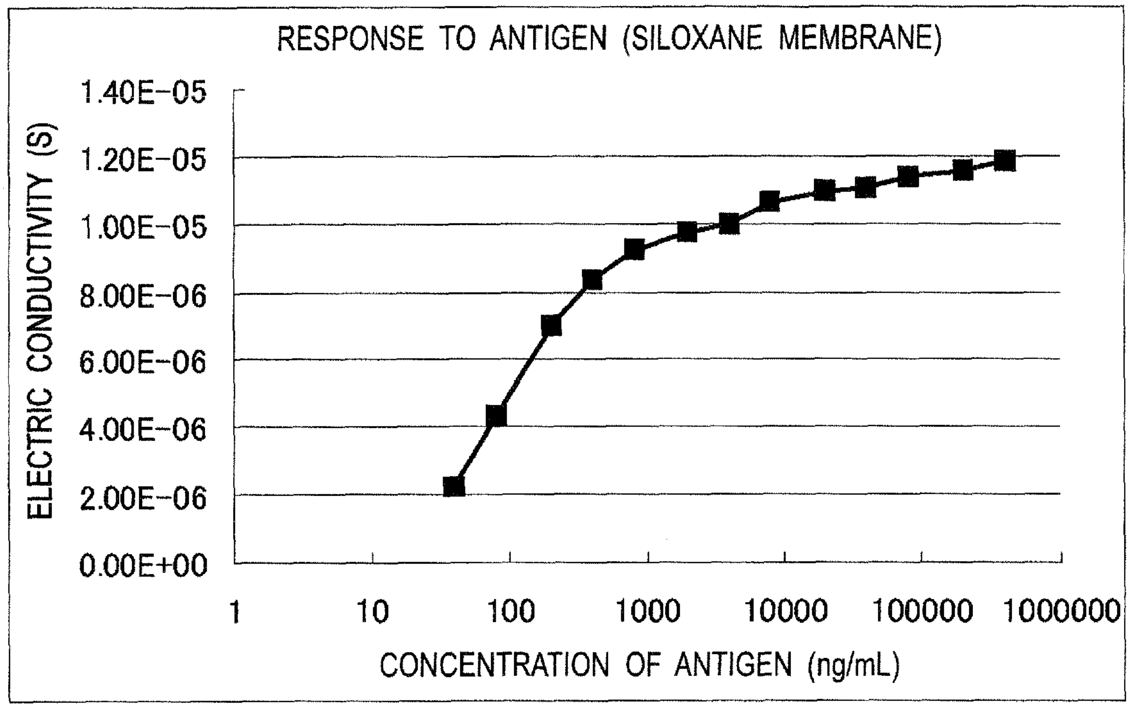
RESPONSE TO ANTIGEN (SILOXANE MEMBRANE)
ELECTRIC CONDUCTIVITY (S)
1.40E-05
1.20E-05
1.00E-05
8.00E-06
6.00E-06
4.00E-06
2.00E-06
0.00E+00
1
10
100
1000
10000
100000
1000000
CONCENTRATION OF ANTIGEN (ng/mL)

*Fig.22*

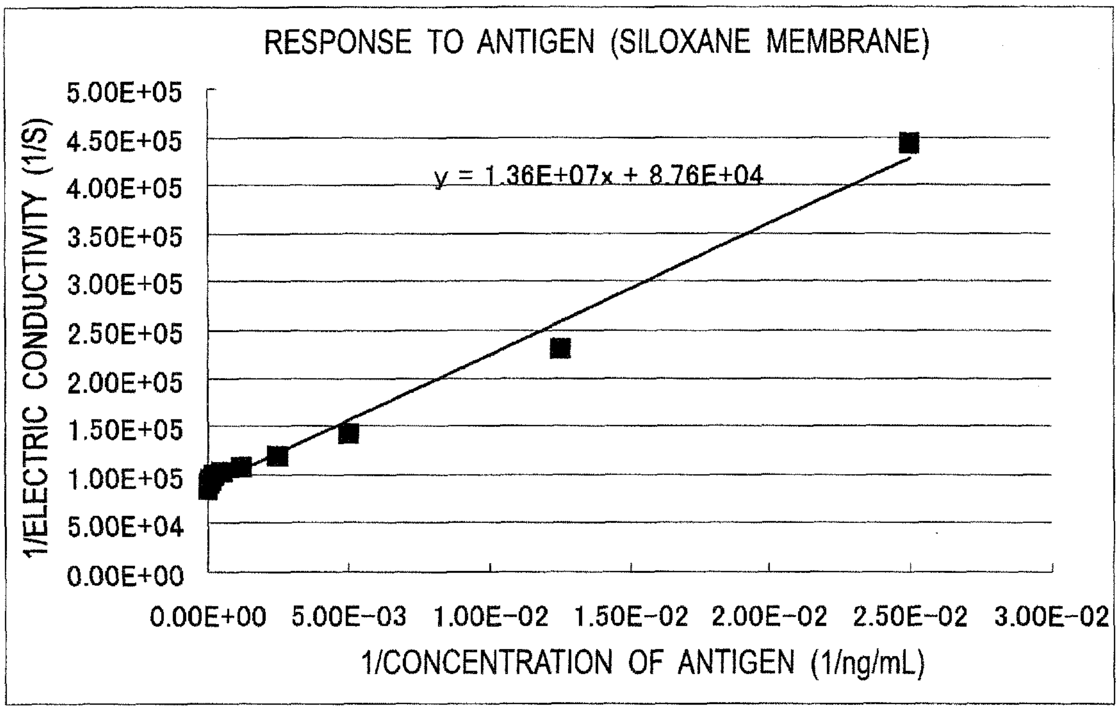
RESPONSE TO ANTIGEN (SILOXANE MEMBRANE)
1/ELECTRIC CONDUCTIVITY (1/S)
5.00E+05
4.50E+05
4.00E+05
3.50E+05
3.00E+05
2.50E+05
2.00E+05
1.50E+05
1.00E+05
5.00E+04
0.00E+00
y = 1.36E+07x + 8.76E+04
0.00E+00
5.00E-03
1.00E-02
1.50E-02
2.00E-02
2.50E-02
3.00E-02
1/CONCENTRATION OF ANTIGEN (1/ng/mL)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002174612 A
- JP 2002333419 A
- JP 11322799 A